# EUROPEAN PATENT APPLICATION

(11) **EP 4 644 539 A1**
(43) Date of publication of application: **05.11.2025**
(21) Application number: 23912332.6
(22) Date of filing: 28.12.2023
(51) Int. Cl.: C12N 5/10, A61K 35/28, A61K 35/44, A61K 48/00, A61P 37/02, A61P 43/00, C12N 5/071, C12N 5/0775, C12N 5/0786, C12N 15/12

(54) **METHOD FOR REGULATING DEGREE OF CELL DIFFERENTIATION**

(30) Priority: 28.12.2022 JP 2022212691
(71) Applicant: National University Corporation Chiba University, Chiba-shi, Chiba 263-8522 (JP)
(72) Inventor: TAKAYAMA, Naoya, Chiba-shi, Chiba 260-8670 (JP); PAUL, Sudip Kumar, Chiba-shi, Chiba 260-8670 (JP); NAKAJIMA, Akihiro, Chiba-shi, Chiba 260-8670 (JP); LIU, YiJing, Chiba-shi, Chiba 260-8670 (JP); NAKAMURA, Sou, Kyoto-shi, Kyoto 606-8501 (JP)
(74) Representative: D Young & Co LLP
(86) International application number: PCT/JP2023/047341
(87) International publication number: WO 2024/143555

(57) **Abstract**

The present invention relates to a method for regulating the degree of cell differentiation including a step of forcibly expressing the MYC family gene and the BMI1 gene in cells selected from the group consisting of (i) mesenchymal stromal cells, vascular endothelial cells, smooth muscle cells, neural crest cells, and lymphocytes having any degree of differentiation, and (ii) megakaryocytes and myelocytes having a high degree of differentiation.

## Description

### Technical Field

The present invention broadly relates to a method for regulating the degree of cell differentiation and the like.

### Background Art

Mesoderm-derived tissues such as bone, blood vessels, and cardiac myocytes differentiate from mesenchymal stromal cells via progenitor cells of each lineage. Mesenchymal stromal cells are somatic stem cells that produce mesoderm-derived tissues such as osteoblasts, adipocytes, and chondrocytes. Mesenchymal stromal cells are present in tissues such as bone marrow, fat, and dental pulp. In addition, peripheral blood cells differentiate from hematopoietic stem cells via hematopoietic progenitor cells of each lineage. Common myeloid progenitor cells are hematopoietic progenitor cells that produce platelets, erythrocytes, and additionally leukocytes other than lymphocytes (neutrophils, macrophages, basophils, dendritic cells, etc.). These cells exist in the bone marrow under normal conditions, but differentiate as necessary and supply mature blood cells in response to external injury or infection. The produced neutrophils, macrophages, basophils, dendritic cells and the like play key roles in innate immunity, and contribute to defense against various pathogenic organisms, elimination of tumors and degenerated self cells, allergic reactions, and acute and chronic inflammation.

These cells are expected to be used as a cell therapy source for screening drugs against inflammation and allergies and for removing foreign substances from the body. In conventional techniques, methods of inducing differentiation in vitro from umbilical cord blood, bone marrow blood, human ES/iPS cells and the like have been used, but all of these methods are complicated, and it is difficult to prepare a large amount of cells.

### Citation List

### Non-Patent Literature

Non-Patent Literature 1: Jie Z, Zhang Y, Wang C, Shen B, Guan X, Ren Z, et al. Large-scale ex vivo generation of human neutrophils from cord blood CD34+ cells. PLoS One. 2017; 12(3).
Non-Patent Literature 2: Caux C, Vanbervliet B, Massacrier C, Dezutter-Dambuyant C, De Saint-Vis B, Jacquet C, et al. CD34+ hematopoietic progenitors from human cord blood differentiate along two independent dendritic cell pathways in response to GM-CSF+TNFα. J Exp Med. 1996; 184(2): 695-706.
Non-Patent Literature 3: Lachmann N, Ackermann M, Frenzel E, Liebhaber S, Brennig S, Happle C, et al. Large-scale hematopoietic differentiation of human induced pluripotent stem cells provides granulocytes or macrophages for cell replacement therapies. Stem Cell Reports. 2015;
Non-Patent Literature 4: Hiramoto T, Ebihara Y, Mizoguchi Y, Nakamura K, Yamaguchi K, Ueno K, et al. Wnt3a stimulates maturation of impaired neutrophils developed from severe congenital neutropenia patient-derived pluripotent stem cells. Proc Natl Acad Sci U S A. 2013; 110(8): 3023-8.
Non-Patent Literature 5: Sweeney CL, Teng R, Wang H, Merling RK, Lee J, Choi U, et al. Molecular Analysis of Neutrophil Differentiation from Human Induced Pluripotent Stem Cells Delineates the Kinetics of Key Regulators of Hematopoiesis. Stem Cells. 2016; 34(6): 1513-26.
Non-Patent Literature 6: Takata K, Kozaki T, Lee CZW, Thion MS, Otsuka M, Lim S, et al. Induced-Pluripotent-Stem-Cell-Derived Primitive Macrophages Provide a Platform for Modeling Tissue-Resident Macrophage Differentiation and Function. Immunity. 2017; 47(1): 183-198.e6.
Non-Patent Literature 7: Cao X, Yakala GK, van den Hil FE, Cochrane A, Mummery CL, Orlova V V. Differentiation and Functional Comparison of Monocytes and Macrophages from hiPSCs with Peripheral Blood Derivatives. Stem Cell Reports. 2019; 12(6): 1282-97.
Non-Patent Literature 8: Ackermann M, Kempf H, Hetzel M, Hesse C, Hashtchin AR, Brinkert K, et al. Bioreactor-based mass production of human iPSC-derived macrophages enables immunotherapies against bacterial airway infections. Nat Commun. 2018; 9(1).
Non-Patent Literature 9: Combination of immortalization and inducible death strategies to generate a human mesenchymal stromal cell line with controlled survival. Bourgine P, Le Magnen C, Pigeot S, Geurts J, Scherberich A, Martin I. Stem Cell Res. 2014 Mar; 12(2): 584-98

### Summary of Invention

### Technical Problem

In methods using hematopoietic progenitor cells derived from umbilical cord blood and bone marrow blood, neutrophils, macrophages and the like can be obtained by adding growth factors (refer to Non-Patent Literature 1 and 2), but proliferation of umbilical cord blood and bone marrow blood itself is limited, there are large differences between lots, and it is difficult to prepare a large amount of cells with uniform quality.

In methods using human ES/iPS cells, ES/iPS cells themselves can proliferate almost indefinitely, but the method of inducing differentiation is complicated, the efficiency of induction into blood cells is low, and it is difficult to supply a large amount at a clinical application level (refer to Non-Patent Literature 3 to 7). For example, in a reported method of differentiating macrophages from human iPS cells, the maximum number of cells is 10⁸ cells in a 250 mL culture system (refer to Non-Patent Literature 8). In consideration of both drug screening and cell therapy, in conventional techniques, the number of cells is significantly insufficient, and the development of more efficient induction methods is essential.

As immortalized cell lines of myeloid lineage, there are cell lines that have been established with a low probability by culturing cells collected from leukemia patients, but these cells express leukemia mutated genes constitutively, normal differentiation is not possible, and drug screening performed using mature cells is not possible. In addition, there is a risk of these cells becoming cancerous, and not being able to be used as a cell therapy source.

As immortalized cell lines of mesenchymal stromal cell lineage, there are cell lines prepared by forcibly expressing hTERT from human bone marrow-derived mesenchymal stromal cells (Non-Patent Literature 9), but this requires bone marrow aspiration, which involves an invasive procedure. The literature does not describe that MSCs at any differentiation stage can be reverted (made into a more primitive state), and does not demonstrate the ability to revert them into progenitor cells.

In view of such circumstances, an object of the present invention is to provide a novel method for regulating cell differentiation in order to establish a stable production system for cells such as mesenchymal stromal cells, vascular endothelial cells, smooth muscle cells, neural crest cells, lymphocytes, neutrophils, macrophages, basophils, and dendritic cells.

### Solution to Problem

The inventors conducted extensive studies in order to achieve the above object, and as a result, found that the degree of cell differentiation can be regulated by forcibly expressing the MYC family gene and the BMI1 gene in mesenchymal stromal cells, vascular endothelial cells, smooth muscle cells, neural crest cells, and lymphocytes having any degree of differentiation, and in megakaryocytes and myelocytes having a high degree of differentiation, and completed the present invention.

Specifically, the present invention includes the following inventions.
[1] A method for regulating the degree of cell differentiation, including a step of forcibly expressing the MYC family gene and the BMI1 gene in cells selected from the group consisting of (i) mesenchymal stromal cells, vascular endothelial cells, smooth muscle cells, neural crest cells, and lymphocytes having any degree of differentiation, and (ii) megakaryocytes and myelocytes having a high degree of differentiation.
[2] The method according to [1], wherein the cells are mesenchymal stromal cells having any degree of differentiation.
[3] The method according to [1] or [2], wherein the cells are mesenchymal stromal cells that do not express a cell surface marker CD90.
[4] The method according to any one of [1] to [3], wherein the cells are mesenchymal stromal cells that express a cell surface marker CD90.
[5] The method according to any one of [1] to [4], wherein the cells are vascular endothelial cells having any degree of differentiation.
[6] The method according to any one of [1] to [5], wherein the cells are smooth muscle cells having any degree of differentiation.
[7] The method according to any one of [1] to [6], wherein the cells are vascular smooth muscle cells having any degree of differentiation.
[8] The method according to any one of [1] to [7], wherein the cells are vascular smooth muscle cells that express a cell surface marker CD140b, KDR, or CD34.
[9] The method according to any one of [1] to [8], wherein the cells are neural crest cells having any degree of differentiation.
[10] The method according to any one of [1] to [9], wherein the cells are lymphocytes having any degree of differentiation.
[11] The method according to any one of [1] to [10], wherein the cells are megakaryocytes that express cell surface markers CD41 and CD42b.
[12] The method according to any one of [1] to [11], wherein the cells are myelocytes that express cell surface markers CD14 and CD11b.
[13] The method according to any one of [1] to [12], further including a step of inhibiting the expression of the MYC family gene and the BMI1 gene or the function of expression products thereof.
[14] The method according to any one of [1] to [13], further including a step of forcibly expressing the BCL-XL gene.
[15] The method according to any one of [1] to [14], further including a step of inhibiting the expression of the BCL-XL gene or the function of expression products thereof.
[16] The method according to any one of [1] to [15], further including a step of inhibiting the expression of at least one of the CDKN1A gene and the P53 gene or the function of expression products thereof.
[17] A method for producing progenitor cells of mesenchymal stromal cells, vascular endothelial cells, smooth muscle cells, neural crest cells, lymphocytes, megakaryocytes, or myelocytes, including a step of culturing cells obtained by the method according to any one of [1] to [16].
[18] A method for producing differentiated cells of mesenchymal stromal cells, vascular endothelial cells, smooth muscle cells, neural crest cells, lymphocytes, megakaryocytes, or myelocytes, including a step of differentiating cells obtained by the method according to any one of [1] to [16].
[19] Cells obtained by the method according to any one of [1] to [18].
[20] A pharmaceutical composition containing cells obtained by the method according to any one of [1] to [18].
[21] A method for treating or preventing a disease, including administering the cells according to [19] or the pharmaceutical composition according to [20] to a patient in need thereof.
[22] A cell differentiation regulating agent for cells selected from the group consisting of (i) mesenchymal stromal cells, vascular endothelial cells, smooth muscle cells, neural crest cells, and lymphocytes having any degree of differentiation, and (ii) megakaryocytes and myelocytes having a high degree of differentiation, including molecules that forcibly express the MYC family gene and the BMI1 gene as an active component.
[23] A method for producing macrophages including the following steps:
   1) a step of forcibly expressing the MYC family gene and the BMI1 gene in myelocytes having a high degree of differentiation,
   2) a step of culturing and proliferating the cells obtained in Step 1, and
   3) a step of inhibiting the forced expression of the MYC family gene and the BMI1 gene in the cells obtained in Step 2, and additionally culturing them under macrophage differentiation conditions to promote differentiation and maturation into macrophages.
[24] The method according to [23], wherein Step 1 further includes forcibly expressing the BCL-XL gene in myelocytes having a high degree of differentiation.
[25] The method according to [23] or [24], wherein Step 3 further includes inhibiting the forced expression of the BCL-XL gene in the cells obtained in Step 2.
[26] The method according to any one of [23] to [25], wherein Step 1 further includes inhibiting the expression of the CDKN1A gene and/or the p53 gene or the function of expression products thereof in myelocytes having a high degree of differentiation.
[27] A method for producing mesenchymal stromal cells having a proliferative ability, including a step of forcibly expressing the MYC family gene and the BMI1 gene in mesenchymal stromal cells having any degree of differentiation.
[28] The production method according to [27], wherein the mesenchymal stromal cells having any degree of differentiation are mesenchymal stromal cells that do not express a cell surface marker CD90.
[29] The production method according to [27] or [28], wherein the mesenchymal stromal cells having any degree of differentiation are mesenchymal stromal cells that express a cell surface marker CD90.
[30] The production method according to any one of [27] to [29], wherein the mesenchymal stromal cells having a proliferative ability express a cell surface marker CD90.
[31] A method for producing vascular endothelial cells having a proliferative ability, including a step of forcibly expressing the MYC family gene and the BMI1 gene in vascular endothelial cells having any degree of differentiation.
[32] A method for producing smooth muscle cells having a proliferative ability, including a step of forcibly expressing the MYC family gene and the BMI1 gene in smooth muscle cells having any degree of differentiation.
[33] The production method according to [32], wherein the smooth muscle cells having any degree of differentiation are vascular smooth muscle cells.
[34] The production method according to [33], wherein the vascular smooth muscle cells are vascular smooth muscle cells that express a cell surface marker CD140b, KDR, or CD34.
[35] A method for producing neural crest cells having a proliferative ability, including a step of forcibly expressing the MYC family gene and the BMI1 gene in neural crest cells having any degree of differentiation.
[36] A method for producing lymphocytes having a proliferative ability, including a step of forcibly expressing the MYC family gene and the BMI1 gene in lymphocytes having any degree of differentiation.
[37] A method for producing megakaryocytes having a proliferative ability, including a step of forcibly expressing the MYC family gene and the BMI1 gene in megakaryocytes that express cell surface markers CD41 and CD42b.
[38] A method for producing macrophages having a proliferative ability, including a step of forcibly expressing the MYC family gene and the BMI1 gene in macrophages that express cell surface markers CD14 and CD11b.
[39] A method for promoting differentiation of iPS cells, ES cells, hematopoietic stem cells or hematopoietic progenitor cells into lymphocyte cells or lymphoid progenitor cells, including a step of co-culturing immortalized vascular endothelial cells, and iPS cells, ES cells, hematopoietic stem cells or hematopoietic progenitor cells.
[40] The method according to [39], wherein the lymphocyte cells express cell surface markers CD45 and CD56 or CD19 and CD45.
[41] The method according to [39] or [40], wherein the lymphocyte cells are natural killer cells or B cells.
[42] The method according to any one of [39] to [41], wherein the hematopoietic stem cells or hematopoietic progenitor cells are derived from umbilical cord blood or iPS cells.
[43] The method according to any one of [39] to [42], wherein immortalized vascular endothelial cells express a Notch ligand.
[44] The method according to [43], wherein the Notch ligand is DLL4 and/or Jagged 1.

### Advantageous Effect of Invention

According to the present invention, the degree of cell differentiation can be regulated by forcibly expressing the MYC family gene and the BMI1 gene in mesenchymal stromal cells, vascular endothelial cells, smooth muscle cells, neural crest cells, and lymphocytes having any degree of differentiation, and in megakaryocytes and myelocytes having a high degree of differentiation.

According to the present invention, it is possible to induce immortalized mesenchymal, megakaryocytic, or myeloid cell lines from various iPS cells with high efficiency. Therefore, the use of iPS cells into which receptors targeting foreign substance-specific antigens are introduced, iPS cells that are genetically modified to enhance cytotoxicity against target cells, HLA null iPS cells that minimize immune rejection reactions, and iPS cells for genetic diseases is more in vivo-like and can be expected to have advantages in both drug screening and cell therapy.

### Brief Description of Drawings

[Figure 1] Figure 1 shows the evaluation results of cell proliferation of mesenchymal stromal cells.
[Figure 2] Figure 2 shows the results of flow cytometry analysis of mesenchymal stromal cells.
[Figure 3] Figure 3 shows the results of flow cytometry analysis when mesenchymal stromal cells were separated into CD90- cells and CD90+ cells, and cultured after MB was forcibly expressed.
[Figure 4] Figure 4 shows the results obtained by comparing cell proliferation of mesenchymal stromal cells when Dox was turned on and when Dox was turned off.
[Figure 5] Figure 5 shows the results of flow cytometry analysis of mesenchymal stromal cells when Dox was turned on and when Dox was turned off.
[Figure 6] Figure 6 shows the results of inducing terminal differentiation of mesenchymal stromal cells.
[Figure 7] Figure 7 shows the evaluation results of cell proliferation of vascular endothelial cells.
[Figure 8] Figure 8 shows the results of flow cytometry analysis of vascular endothelial cells.
[Figure 9] Figure 9 shows the results of morphological observation of vascular endothelial cells.
[Figure 10] Figure 10 shows the results of immunostaining of vascular endothelial cells.
[Figure 11] Figure 11 shows the results of marker expression of vascular endothelial cells.
[Figure 12] Figure 12 shows the evaluation results of cell proliferation of vascular smooth muscle cells.
[Figure 13] Figure 13 shows the results of immunostaining of vascular smooth muscle cells.
[Figure 14] Figure 14 is a schematic diagram showing an experiment in which vascular smooth muscle cells are reprogrammed into progenitor cells.
[Figure 15] Figure 15 shows the results of morphological observation of vascular smooth muscle cells in the experiment of Figure 14.
[Figure 16] Figure 16 shows the evaluation results of cell proliferation of vascular smooth muscle cells in the experiment of Figure 14.
[Figure 17-1] Figure 17-1 shows the results of flow cytometry analysis (CD140b) of vascular smooth muscle cells in the experiment of Figure 14.
[Figure 17-2] Figure 17-2 shows the results of flow cytometry analysis (KDR) of vascular smooth muscle cells in the experiment of Figure 14.
[Figure 17-3] Figure 17-3 shows the results of flow cytometry analysis (CD34) of vascular smooth muscle cells in the experiment of Figure 14.
[Figure 18] Figure 18 shows the results of immunostaining of vascular smooth muscle cells in the experiment of Figure 14.
[Figure 19] Figure 19 shows the results of morphological observation of neural crest cells.
[Figure 20] Figure 20 shows the evaluation results of cell proliferation of neural crest cells.
[Figure 21] Figure 21 shows the results of flow cytometry analysis (CD271) of neural crest cells.
[Figure 22] Figure 22 shows the results of flow cytometry analysis (CD271) of neural crest cells.
[Figure 23] Figure 23 shows the evaluation results of the effect of fibronectin coating on neural crest cells.
[Figure 24] Figure 24 shows the evaluation results of cell proliferation of CD45+/CD56+ cells.
[Figure 25] Figure 25 shows the results of flow cytometry analysis of CD45+/CD56+ cells.
[Figure 26] Figure 26 shows the evaluation results of cell proliferation of CD45+/CD19+ cells.
[Figure 27] Figure 27 shows the results of flow cytometry analysis of CD45+/CD19+ cells.
[Figure 28] Figure 28 shows the evaluation results of cell proliferation of megakaryocytes and myelocytes (macrophages) and the results of flow cytometry analysis.
[Figure 29] Figure 29 shows the results of principal component analysis (PCA) of dox-on immortalized vascular endothelial cells and dox-off immortalized vascular endothelial cells, human iPS cells, human aortic endothelial cells (HAEC), and human umbilical vein endothelial cells (HUVEC).
[Figure 30] Figure 30 shows the results of expression analysis of DLL4 and Jagged 1 in dox-on immortalized vascular endothelial cells, and dox-off immortalized vascular endothelial cells, human iPS cells, human aortic endothelial cells (HAEC), and human umbilical vein endothelial cells (HUVEC).
[Figure 31] Figure 31 shows the results of flow cytometry analysis of CD45+/CD56+ cells when immortalized vascular endothelial cells and CD34-positive umbilical cord blood cells were co-cultured.
[Figure 32] Figure 32 shows the evaluation results of cell proliferation of CD45+/CD56+ cells when immortalized vascular endothelial cells and CD34-positive umbilical cord blood cells were co-cultured.
[Figure 33] Figure 33 shows the results of flow cytometry analysis of CD45+/CD19+ cells when iPS cell-derived human hematopoietic stem cells were co-cultured with immortalized vascular endothelial cells or gene recombinant DLL4.
[Figure 34] Figure 34 shows the results of flow cytometry analysis of CD45+/CD56+ cells and CD45+/CD19+ cells when immortalized vascular endothelial cells and iPS cell-derived human hematopoietic stem cells were co-cultured after IL-3 was administered.

### Description of Embodiments

A method for regulating the degree of cell differentiation according to the present embodiment includes a step of changing the expression levels of the MYC family gene and the BMI1 gene (for example, forcibly expressing the MYC family gene and the BMI1 gene and inhibiting the expression of the MYC family gene and the BMI1 gene) in mesenchymal stromal cells, vascular endothelial cells, smooth muscle cells, neural crest cells, and lymphocytes having any degree of differentiation, and in megakaryocytes and myelocytes having a high degree of differentiation. When the MYC family gene and the BMI1 gene are forcibly expressed in mesenchymal stromal cells, vascular endothelial cells, smooth muscle cells, neural crest cells, and lymphocytes having any degree of differentiation, and megakaryocytes and myelocytes having a high degree of differentiation, cells having a low degree of differentiation (mesenchymal stromal cells, vascular endothelial cells, smooth muscle cells, neural crest cells, lymphocytes, megakaryocytes, and myelocytes) can be obtained compared to before forced expression. When the MYC family gene and the BMI1 gene are forcibly expressed in mesenchymal stromal cells, vascular endothelial cells, smooth muscle cells, neural crest cells, lymphocytes, megakaryocytes, and myelocytes having a high degree of differentiation, cells having a low degree of differentiation (mesenchymal stromal cells, vascular endothelial cells, smooth muscle cells, neural crest cells, lymphocytes, megakaryocytes, and myelocytes) can be obtained compared to before forced expression. The cells having a low degree of differentiation can have a proliferative ability. In addition, when the expression of the MYC family gene and the BMI1 gene in the cells having a low degree of differentiation (mesenchymal stromal cells, vascular endothelial cells, smooth muscle cells, neural crest cells, lymphocytes, megakaryocytes, and myelocytes) obtained by the forced expression of the MYC family gene and the BMI1 gene is inhibited, cells having a high degree of differentiation (mesenchymal stromal cells, vascular endothelial cells, smooth muscle cells, neural crest cells, lymphocytes, megakaryocytes, and myelocytes) can be obtained compared to before expression inhibition.

The term "degree of differentiation" or "degree of cell differentiation" refers to the level or extent to which cells differentiate or the degree of maturity.

The degree of cell differentiation may be determined based on the expression level or extent of cell surface markers. For example, the degree of differentiation may be determined by detecting the expression level of cell surface markers expressed by cells.

The degree of cell differentiation may be determined relatively. For example, in a differentiation process of certain cells, when there are cells having a certain degree of differentiation and cells having a different degree of differentiation from those cells, the degrees of differentiation of both groups of cells may be compared to determine whether cells have a relatively low degree of differentiation or cells have a relatively high degree of differentiation. When the degree of differentiation of cells is determined relatively, the degree of differentiation may be determined based on the amount or extent of expression of cell surface markers.

In the present embodiment, cells "having any degree of differentiation" or "having a high degree of differentiation" may be produced by induction from pluripotent stem cells such as iPS cells and ES cells, or may be produced by the method for regulating the degree of cell differentiation of the present invention.

In the present embodiment, the regulation of the degree of cell differentiation may be an improvement in cell proliferation (proliferative ability) or a decrease in cell proliferation or may include both an improvement and a decrease in cell proliferation. That is, the decrease in cell proliferation may be a regulation of the degree of cell differentiation to a high degree of differentiation, and the improvement in cell proliferation may be a regulation of the degree of cell differentiation to a low degree of differentiation.

### 1-1. Mesenchymal stromal cells (MSC)

The term "mesenchymal stromal cells" refers to stem cells having an ability to differentiate into mesenchymal cells. The mesenchymal stromal cells may include adipose stromal cells and adipose stem cells.

Mesenchymal stromal cells may be obtained by inducing differentiation from pluripotent stem cells such as iPS cells and ES cells by methods well known to those skilled in the art. For example, according to the report of Fukuta et al. (PlosOne, 2014, 9(12): e112291), iPS cells are cultured in a Matrigel-coated culture dish in the presence of SB-431542 and CHIR99021, neural crest cells (NCCs) are induced, the NCCs are cultured in a fibronectin-coated culture dish in a medium containing EGF, FGF2 and SB-431542, the NCCs are amplified, the NCCs are additionally cultured in a fibronectin-coated culture dish in a medium containing 10% FBS and FGF2, and thus MSCs can be induced.

The induction of mesenchymal stromal cells can be confirmed by subjecting the cultured cells to flow cytometry analysis to detect the appearance of cells exhibiting a characteristic cell surface marker expression pattern of mesenchymal stromal cells to be described below or by subjecting the cultured cells to a colony formation assay to confirm that the cells have a differentiation ability of mesenchymal stromal cells.

Mesenchymal stromal cells can differentiate into vascular endothelial cells, osteoblasts, adipocytes, chondrocytes, fibroblasts, myoblasts, bone marrow stromal cells, tendon cells, hepatocytes, bile duct epithelial cells, glia cells, nerve cells, myocardial cells, smooth muscle cells, lymphatic endothelial cells and the like.

In the present embodiment, "progenitor cells of mesenchymal stromal cells" may be progenitor cells of cells selected from the group consisting of bone vascular endothelial cells, osteoblasts, adipocytes, chondrocytes, fibroblasts, myoblasts, bone marrow stromal cells, tendon cells, hepatocytes, bile duct epithelial cells, glia cells, nerve cells, myocardial cells, smooth muscle cells, and lymphatic endothelial cells.

In the present embodiment, "differentiated cells of mesenchymal stromal cells" may be cells selected from the group consisting of vascular endothelial cells, osteoblasts, adipocytes, chondrocytes, fibroblasts, myoblasts, bone marrow stromal cells, tendon cells, hepatocytes, bile duct epithelial cells, glia cells, nerve cells, myocardial cells, smooth muscle cells, and lymphatic endothelial cells.

Mesenchymal stromal cells can be characterized by expressing, for example, cell surface markers CD90, CD73, and CD105.

The degree of differentiation of mesenchymal stromal cells can be characterized by the cell surface marker expression pattern in flow cytometry analysis. For example, when the degree of differentiation of mesenchymal stromal cells increases, the expression level of the cell surface marker CD90 decreases. That is, mesenchymal stromal cells having a high degree of differentiation may not express the cell surface marker CD90 but may express the cell surface markers CD73 and CD105. In one aspect, the mesenchymal stromal cells having a high degree of differentiation are CD90-, CD90-/CD73+, or CD90-/CD105+ mesenchymal stromal cells in flow cytometry analysis. When mesenchymal stromal cells are established from pluripotent stem cells such as iPS cells, they gradually differentiate and their stemness (proliferation) decreases as they are repeatedly passaged, and after 10 passages, mesenchymal stromal cells having a high degree of differentiation (CD90-/CD73+, or CD90-/CD105+) may account for about 90% of live cells. In addition, when the degree of differentiation of mesenchymal stromal cells decreases, the expression level of the cell surface marker CD90 increases. That is, mesenchymal stromal cells having a low degree of differentiation express the cell surface marker CD90 and may express the cell surface markers CD73 and CD105. In one aspect, the mesenchymal stromal cells having a low degree of differentiation are CD90+, CD90+/CD73+, or CD90+/CD105+ mesenchymal stromal cells in flow cytometry analysis. In another aspect, the mesenchymal stromal cells having a low degree of differentiation may be mesenchymal stromal cells in which the CD90-/CD73+ or CD90-/CD105+ fraction is not detected in 10% or more of live cells in flow cytometry analysis.

### 1-2. Vascular endothelial cells

The term "vascular endothelial cells" refers to cells that constitute vascular endothelium or cells that can differentiate into such cells. Among vascular endothelial cells, fetal cells that have an ability to differentiate into blood cells are called hemogenic endothelial cells.

Regarding the hematopoietic mechanism in vivo, in addition to the hematopoietic mechanism by which blood cells derived from stem cells are supplied, a hematopoietic mechanism by which blood cells are supplied through development of vascular endothelial cells is known.

Vascular endothelial cells may be VE-cadherin (VE-cad)-positive, CD41-positive, or CXCR4-positive cells. Vascular endothelial cells may be, for example, cells derived from pluripotent stem cells such as ES cells or iPS cells, or induced during the process of inducing a net-like structure from ES cells or iPS cells.

Cell culture conditions suitable for preparing a net-like structure from human pluripotent stem cells such as human ES cells and human iPS cells vary depending on the pluripotent stem cells used. For example, an IMDM supplemented with FBS at a final concentration of 15% is used as the medium, and even in the case of other serum-free mediums, a medium supplemented with appropriately growth factors, supplements and the like can be used. In addition, in order to efficiently form a net-like structure, 0 to 100 ng/ml of VEGF may be added, and more preferably about 20 ng/ml of VEGF is added. The culture environment varies depending on the type of ES cells or iPS cells used, and for example, conditions of 5% CO₂, 36 to 38°C, and preferably 37°C can be used. The culture period required for the formation of the net-like structure varies depending on the type of pluripotent stem cells and induction conditions, and generally, after pluripotent stem cells are seeded on feeder cells (for example, 10T1/2 cells), cell masses containing hematopoietic endothelial cells are formed within about 7 days, and a net-like structure containing vascular endothelial progenitor cells and hematopoietic progenitor cells is formed within about 14 to 16 days.

The formed net-like structure exhibits a follicular structure and concentrated vascular endothelial progenitor cells and hematopoietic progenitor cells are present therein. The hematopoietic endothelial cells contained in the cell masses and the vascular endothelial progenitor cells and hematopoietic progenitor cells present inside the net-like structure can be separated by passing them through a physical means, for example, a sterilized sieve device (for example, a cell strainer). Then, CD34+ vascular endothelial progenitor cells are isolated using a cell sorter, the cells are continuously cultured in the presence of VEGF, and thus vascular endothelial cells can be obtained.

The induction of vascular endothelial cells can be confirmed by subjecting the cultured cells to flow cytometry analysis to detect the appearance of cells exhibiting a characteristic cell surface marker expression pattern of vascular endothelial cells to be described below or by subjecting the cultured cells to a colony formation assay to confirm that the cells have a differentiation ability of vascular endothelial cells.

Vascular endothelial cells can be characterized by expressing, for example, cell surface markers such as VE-cad, CD31(PECAM1), CD105, and CD146.

The degree of differentiation of vascular endothelial cells can be characterized by the cell surface marker expression pattern in flow cytometry analysis. For example, when the degree of differentiation of vascular endothelial cells increases, the expression level of the cell surface markers such as VE-cad, CD31, CD105, and CD146 increases. That is, the vascular endothelial cells having a high degree of differentiation may express the cell surface markers such as VE-cad, CD31, CD105, and CD146. In one aspect, the vascular endothelial cells having a high degree of differentiation may be VE-cad+/CD31+ vascular endothelial cells in flow cytometry analysis, and may further express one or two cell surface markers selected from the group consisting of CD105 and CD146, and preferably VE-cad+/CD31+/CD105+/CD146+. In addition, when the degree of differentiation of vascular endothelial cells decreases, the expression level of the cell surface markers such as VE-cad, CD31, CD105, and CD146 decreases. That is, vascular endothelial cells having a low degree of differentiation may not express one, two, three or four cell surface markers selected from the group consisting of VE-cad, CD31, CD105 and CD146. In one aspect, vascular endothelial cells having a low degree of differentiation may express VE-cad+/CD31+ in flow cytometry analysis or may be vascular endothelial cells that do not express one or two cell surface markers selected from the group consisting of CD105 and CD146.

### 1-3. Smooth muscle cells

The term "smooth muscle cells" refers to cells that constitute smooth muscle or cells that can differentiate into such cells (for example, smooth muscle progenitor cells, smooth muscle stem cells, etc.). In addition, "smooth muscle cells" may be, for example, vascular smooth muscle cells, digestive tract smooth muscle cells, bladder smooth muscle cells, uterine smooth muscle cells and the like.

Among smooth muscle cells, vascular smooth muscle cells may be obtained by inducing differentiation from pluripotent stem cells such as iPS cells and ES cells of mammals such as humans and are induced during the process of inducing a net-like structure from ES cells or iPS cells. CD34-/VEGFR(KDR)+vascular smooth muscle progenitor cells contained in the net-like structure are isolated using a cell sorter, continuous culture proceeds, and thus vascular smooth muscle cells can be obtained. The fact that vascular smooth muscle cells have been obtained can be confirmed by subjecting the cultured cells to flow cytometry analysis to detect the appearance of cells exhibiting a characteristic cell surface marker expression pattern of vascular smooth muscle cells to be described below or by subjecting the cultured cells to a colony formation assay to confirm that the cells have a differentiation ability of vascular endothelial cells.

Smooth muscle cells can be derived from mesenchymal stromal cells, neural crest cells, bone marrow cells and the like. As smooth muscle cells, differentiated smooth muscle cells and dedifferentiated smooth muscle cells are known. Differentiated smooth muscle cells and dedifferentiated smooth muscle cells can be confirmed by gene expression patterns well known to those skilled in the art.

In the present embodiment, "progenitor cells of smooth muscle cells" may be progenitor cells of the mesenchymal stromal cells. In the present embodiment, "differentiated cells of smooth muscle cells" may be differentiated smooth muscle cells or dedifferentiated smooth muscle cells.

Smooth muscle cells can be characterized by expressing, for example, markers VEGFR(KDR), Calponin, and αSMA.

The degree of differentiation of smooth muscle cells can be characterized by the above marker expression pattern. For example, when the degree of differentiation of vascular smooth muscle cells increases, the expression level of the markers such as Calponin and αSMA increases. That is, vascular smooth muscle cells having a high degree of differentiation may express markers such as Calponin and αSMA. In one aspect, the vascular smooth muscle cells having a high degree of differentiation may be Calponin+/αSMA+ vascular smooth muscle cells in flow cytometry analysis, or may further express markers Calponin and/or αSMA.

In addition to the above markers, smooth muscle cells can be characterized by expressing cell surface markers well known to those skilled in the art, and among smooth muscle cells, vascular smooth muscle cells can be characterized by expressing, for example, cell surface markers such as CD140b, KDR, and CD34.

The degree of differentiation of vascular smooth muscle cells can be characterized by the cell surface marker expression pattern in flow cytometry analysis. For example, when the degree of differentiation of vascular smooth muscle cells increases, the expression level of the cell surface markers such as CD140b, KDR, and CD34 increases. That is, vascular smooth muscle cells having a high degree of differentiation may express the cell surface markers such as CD140b, KDR, and CD34. In one aspect, vascular smooth muscle cells having a high degree of differentiation may further express one, two, or three cell surface markers selected from the group consisting of CD140b, KDR, and CD34 in flow cytometry analysis. In addition, when the degree of differentiation of vascular smooth muscle cells decreases, the expression level of the cell surface markers such as CD140b, KDR, and CD34 decreases. That is, vascular smooth muscle cells having a low degree of differentiation may not express one, two, or three cell surface markers selected from the group consisting of CD140b, KDR, and CD34. In one aspect, vascular smooth muscle cells having a low degree of differentiation may be vascular smooth muscle cells that do not express one, two, or three cell surface markers selected from the group consisting of CD140b, KDR, and CD34 in flow cytometry analysis.

### 1-4. Neural crest cells

Neural crest cells are migratory stem cells, and are cells derived from a population of cells called a neural crest that appears temporarily during development.

Neural crest cells may be obtained by inducing differentiation from pluripotent stem cells such as iPS cells and ES cells by methods well known to those skilled in the art. For example, according to the report of Fukuta et al. (PlosOne, 2014, 9(12): e112291), iPS cells are cultured in a Matrigel-coated culture dish in the presence of an ALK5 inhibitor such as SB-431542 and a GSK3 inhibitor such as CHIR99021, and thus neural crest cells (NCCs) can be induced. In addition, in culturing neural crest cells, in order to promote cell fixation and growth, culture dishes, wells and the like may be coated with fibronectin. In culturing neural crest cells, in order to promote cell growth, growth factors such as EGF and FGF2 may be added to the medium. In one aspect, neural crest cells are cultured in a medium containing an ALK5 inhibitor such as SB-431542, and EGF and FGF2.

The induction of neural crest cells can be confirmed by subjecting the cultured cells to flow cytometry analysis to detect the appearance of cells exhibiting a characteristic cell surface marker expression pattern of neural crest cells to be described below or by subjecting the cultured cells to a colony formation assay to confirm that the cells have a differentiation ability of neural crest cells.

Neural crest cells can differentiate into nerve cells, osteoblasts, chondrocytes, myoblasts, Schwann cells, glia cells, teeth, skeletal muscle cells, smooth muscle cells, melanocytes and the like.

In the present embodiment, "progenitor cells of neural crest cells" may be progenitor cells of cells selected from the group consisting of nerve cells, osteoblasts, chondrocytes, myoblasts, Schwann cells, glia cells, teeth, skeletal muscle cells, smooth muscle cells, and melanocytes.

Neural crest cells can be characterized by expressing, for example, cell surface markers such as CD271.

The degree of differentiation of neural crest cells can be characterized by the cell surface marker expression pattern in flow cytometry analysis. For example, when the degree of differentiation of neural crest cells increases, the expression level of the cell surface markers such as CD271 decreases. That is, neural crest cells having a high degree of differentiation may be neural crest cells that do not express the cell surface markers such as CD271. In addition, when the degree of differentiation of neural crest cells decreases, the expression level of the cell surface markers such as CD271 increases. That is, neural crest cells having a low degree of differentiation may be neural crest cells that express the cell surface markers such as CD271 or may be neural crest cells that express a higher level of the cell surface markers such as CD271 than neural crest cells having a high degree of differentiation.

During culture, the presence of a fibronectin coating on culture dishes, wells and the like may not affect proliferation of neural crest cells or the expression of the cell surface markers such as CD271.

### 1-5. Lymphocytes

Lymphocytes are a type of leukocytes and can be classified into natural killer cells, B cells, T cells and the like.

Lymphocytes can be obtained by inducing differentiation from pluripotent stem cells such as iPS cells and ES cells, hematopoietic stem cells, hematopoietic progenitor cells and the like by methods well known to those skilled in the art. When inducing lymphocyte differentiation, it is possible to induce lymphocyte differentiation with higher efficiency by co-culturing immortalized vascular endothelial cells as feeder cells with pluripotent stem cells, hematopoietic stem cells, hematopoietic progenitor cells and the like and by coating pluripotent stem cells, hematopoietic stem cells, hematopoietic progenitor cells and the like with Notch ligands such as DLL1, DLL4, and Jagged 1. In addition, it is preferable to induce lymphocyte differentiation in the presence of cytokines such as IL7, IL15, IL2, and IL7.

Therefore, the present embodiment provides a method for promoting differentiation of pluripotent stem cells such as iPS cells and ES cells, hematopoietic stem cells or hematopoietic progenitor cells into lymphocyte cells or lymphoid progenitor cells, including a step of co-culturing immortalized vascular endothelial cells, and pluripotent stem cells such as iPS cells and ES cells, hematopoietic stem cells or hematopoietic progenitor cells.

The immortalized vascular endothelial cells may be vascular endothelial cells having a proliferative ability that are obtained by forcibly expressing the MYC family gene (for example, the c-Myc gene) and the BMI1 gene (and, optionally the BCL-X1 gene) in vascular endothelial cells in the method for regulating the degree of cell differentiation of the present invention. The vascular endothelial cells having a proliferative ability may be cells having a low degree of differentiation compared to before forced expression. Mature vascular endothelial cells obtained by inducing differentiation by stopping the forced expression of the MYC family gene (for example, the c-Myc gene) and the BMI1 gene (and, optionally the BCL-XL gene) in the vascular endothelial cells having a proliferative ability are also included in the immortalized vascular endothelial cells.

It is known that a Notch signal, which is transmitted through the interaction between Notch and Notch ligands (Delta-like4 (hereinafter referred to as DLL4), Jagged 1, etc.), is necessary for cell differentiation, and the immortalized vascular endothelial cells used in the co-culture may express Notch ligands, for example, DLL4 and/or Jagged 1. When immortalized vascular endothelial cells express DLL4 and/or Jagged 1 necessary for notch signal transmission, differentiation of pluripotent stem cells such as iPS cells and ES cells, hematopoietic stem cells or hematopoietic progenitor cells into lymphocyte cells or lymphoid progenitor cells can be promoted. Since differentiation into a wide variety of cells can be promoted when immortalized vascular endothelial cells express DLL4 and/or Jagged 1, the method of the present embodiment may be a method for promoting differentiation of pluripotent stem cells such as iPS cells and ES cells, hematopoietic stem cells or hematopoietic progenitor cells into arbitrary cells, including a step of co-culturing immortalized vascular endothelial cells, and pluripotent stem cells such as iPS cells and ES cells, hematopoietic stem cells or hematopoietic progenitor cells.

The pluripotent stem cells such as iPS cells and ES cells, hematopoietic stem cells or hematopoietic progenitor cells used in the co-culture may be those in which expression cassettes of desired genes (for example, the MYC family gene and the BMI1 gene, optionally additionally the BCL-XL gene) are incorporated in advance, and differentiation of iPS cells, ES cells, hematopoietic stem cells or hematopoietic progenitor cell into target cells may be induced while the genes are forcibly expressed. In this case, differentiation of immortalized target cells can be induced.

The hematopoietic stem cells or hematopoietic progenitor cells used in the co-culture may be those collected from an organism, for example, derived from umbilical cord blood, bone marrow, or peripheral blood, or may be those induced from pluripotent stem cells such as iPS cells.

Since the net-like structure induced from pluripotent stem cells such as iPS cells contains hematopoietic progenitor cells, the net-like structure may be subjected to co-culture as hematopoietic stem cells or hematopoietic progenitor cells.

The immortalized vascular endothelial cells used in the co-culture may be cells that become immortalized by regulating the degree of differentiation in the method for regulating the degree of cell differentiation according to the present embodiment. In addition, the immortalized vascular endothelial cells used in the co-culture may be cells that are treated with antibiotic substances such as mitomycin C or radiation exposure using gamma rays or the like in order to prevent proliferation or may be untreated cells.

Co-culture conditions can be appropriately determined by those skilled in the art depending on the state of pluripotent stem cells such as iPS cells and ES cells, hematopoietic stem cells or hematopoietic progenitor cells, and immortalized vascular endothelial cells. For example, the culture temperature can be about 35°C to about 42°C, about 36°C to about 40°C, or about 37°C to about 39°C, the carbon dioxide concentration can be, for example, 5% CO₂, and the oxygen concentration can be, for example, 20% O₂. The culture may be stationary culture or shaking culture. The shaking speed in the case of shaking culture is not particularly limited, and can be, for example, 10 rpm to 200 rpm, or 30 rpm to 150 rpm. The medium may be an Iscove's modified Dulbecco's medium (IMDM) containing serum, insulin, transferrin, serine, thioglycerol, ascorbic acid, and TPO. In this case, the IMDM medium may further contain SCF and may further contain heparin. In addition, phorbol esters (for example, phorbol-12-myristate-13-acetate; PMA) may be added. In addition, the medium may contain serum or plasma or may be serum-free. When serum is used, human serum is preferable. As necessary, for example, the medium may also contain one or more substances such as albumin, insulin, transferrin, selenium, fatty acids, trace elements, 2-mercaptoethanol, thioglycerol, monothioglycerol (MTG), lipids, amino acids (for example, L-glutamine), ascorbic acid, heparin, non-essential amino acids, vitamins, growth factors, low-molecular-weight compounds, antibiotic substance, antioxidants, pyruvate, buffer agents, inorganic salts, and cytokines. Examples of cytokines include vascular endothelial growth factors (VEGF), thrombopoietin (TPO), various TPO-like substances, stem cell factors (SCF), erythropoietin (EPO), granulocyte colony-stimulating factors (G-CSF), interleukin 3(IL3), ITS (insulin-transferrin-selenite) supplements, and ADAM (A Disintegrin And Metalloprotease) inhibitors.

In one aspect, a combination containing FIt3L, SCF, IL-7 and IL-15 is added as cytokines. The combination may further contain IL-3. In one aspect, in a medium containing IL-3, FIt3L, SCF, IL-7 and IL-15, immortalized vascular endothelial cells, and pluripotent stem cells such as iPS cells and ES cells, hematopoietic stem cells or hematopoietic progenitor cells are co-cultured, and co-culture is then continued in a medium containing FIt3L, SCF, IL-7 and IL-15. The medium for continued culture may not contain IL-3.

In addition, the order of adding immortalized vascular endothelial cells and pluripotent stem cells such as iPS cells and ES cells, hematopoietic stem cells or hematopoietic progenitor cells to a medium may be arbitrary, and immortalized vascular endothelial cells may be added first or pluripotent stem cells such as iPS cells and ES cells, hematopoietic stem cells or hematopoietic progenitor cells may be added first. Co-culture may be performed by adding pluripotent stem cells such as iPS cells and ES cells, hematopoietic stem cells or hematopoietic progenitor cells to immortalized vascular endothelial cells cultured in a medium, or co-culture may be performed by adding immortalized vascular endothelial cells to pluripotent stem cells such as iPS cells and ES cells, hematopoietic stem cells or hematopoietic progenitor cells cultured in a medium. The co-culture may be a two-dimensional culture in a well plate or the like or a three-dimensional culture in an environment close to that in a living body.

By co-culturing with immortalized vascular endothelial cells, it is possible to induce differentiation into target cells with high efficiency, compared to when the cells are not co-cultured with immortalized vascular endothelial cells or when the cells are co-cultured with genetically mutant DLL4. That is, when the cells are co-cultured with immortalized vascular endothelial cells, it is possible to induce differentiation into a larger number and/or proportion of target cells.

The induction of lymphocyte cells or lymphoid progenitor cells can be confirmed by subjecting the cultured cells to flow cytometry analysis to detect the appearance of cells exhibiting a characteristic cell surface marker expression pattern of lymphocytes to be described below or by subjecting the cultured cells to a colony formation assay to confirm that the cells have a differentiation ability of lymphocytes.

Lymphocytes can be derived from hematopoietic stem cells, lymphoid progenitor cells and the like.

In the present embodiment, "lymphoid progenitor cells" may be progenitor cells of cells selected from the group consisting of natural killer cells, B cells, and T cells.

Lymphocytes can be characterized by expressing, for example, cell surface markers such as CD45, CD56, and CD19. CD45 is a cell surface marker that is expressed on all blood cells except for erythrocytes and platelets, CD56 is a characteristic cell surface marker for natural killer cells, and CD19 is a characteristic cell surface marker for B cells. For example, natural killer cells are characterized by expressing CD45 and CD56, and B cells are characterized by expressing CD45 and CD19.

The degree of differentiation of lymphocytes can be characterized by the cell surface marker expression pattern in flow cytometry analysis. For example, when the degree of differentiation of lymphocytes increases, the expression level of the cell surface markers such as CD45, CD56, and CD19 decreases. That is, when the degree of differentiation of lymphocytes increases, in flow cytometry analysis, the expression of the cell surface markers CD45 and CD56 may not be confirmed, the expression of the cell surface markers CD45 and CD19 may not be confirmed, or the expression of the cell surface markers CD45, CD56 and CD19 may not be confirmed. In addition, when the degree of differentiation of lymphocytes decreases, the expression level of the cell surface markers such as CD45, CD56, and CD19 increases. That is, when the degree of differentiation of lymphocytes decreases, the number of cells expressing one, two, or three cell surface markers selected from the group consisting of CD45, CD56, and CD19 increases.

### 1-6. Megakaryocytes

The term "megakaryocytes" refers to cells that can produce platelets, and may also be called megakaryocyte-erythrocyte progenitor( MEP). Megakaryocytes may be CD41a-positive, CD41b-positive, or CD42b-positive cells.

Megakaryocytes may be obtained by inducing differentiation from pluripotent stem cells such as iPS cells and ES cells by methods well known to those skilled in the art. For example, hematopoietic progenitor cells contained in the net-like structure induced from pluripotent stem cells such as human ES cells and iPS cells are isolated using a cell sorter and cultured under megakaryocyte induction conditions (on feeder cells, in the presence of TPO, SCF and Heparin), and thus megakaryocytes can be obtained (WO 2008/041370, Takayama Blood 2008, 111(11): 5298-5306). Alternatively, MYC and BMI1 are forcibly expressed in hematopoietic progenitor cells contained in the net-like structure induced from pluripotent stem cells such as human ES cells and iPS cells and the cells are cultured under megakaryocyte induction conditions (on feeder cells, in the presence of TPO, SCF and Heparin), and thus immortalized megakaryocytes are obtained, and when the expression of MYC and BMI1 in the immortalized megakaryocytes is stopped, mature megakaryocytes can be obtained (WO 2011/034073).

The induction of megakaryocytes can be confirmed by subjecting the cells to a colony formation assay to confirm that the cells have a differentiation ability of megakaryocytes.

Megakaryocytes are a type of blood cells and can be derived from bone marrow cells (hematopoietic stem cells). Hematopoietic stem cells can differentiate into hematopoietic progenitor cells, and hematopoietic progenitor cells can differentiate into blood cells such as erythrocytes, granulocytes, monocytes, macrophages, megakaryocytes and platelets. Examples of granulocytes include myelocytes, promyelocytes, metamyelocytes, myeloblasts, band cells, and segmented cells (neutrophils, eosinophils, basophils, etc.).

In the present embodiment, "progenitor cells of megakaryocytes" may be megakaryocyte-erythroid progenitor cells (MEP).

In the present embodiment, "differentiated cells of megakaryocytes" may be megakaryocytes, platelets and the like.

Megakaryocytes can be characterized by expressing, for example, the cell surface markers CD41 and CD42b, in flow cytometry analysis.

The degree of differentiation of megakaryocytes can be characterized by the cell surface marker expression pattern in flow cytometry analysis. For example, when the degree of differentiation of megakaryocytes increases, the expression level of the cell surface markers CD41 and CD42b increases. That is, megakaryocytes having a high degree of differentiation may express the cell surface markers CD41 and CD42b. Megakaryocytes having a high degree of differentiation may be CD41+/CD42b+ megakaryocytes in flow cytometry analysis. In addition, when the degree of differentiation of megakaryocytes decreases, the expression level of the cell surface marker CD42b decreases. That is, megakaryocytes having a low degree of differentiation may express CD41+, but may not express the cell surface marker CD42b. The megakaryocytes having a low degree of differentiation may be CD41+/CD42b- megakaryocytes.

### 1-7. Myelocytes

The term "myelocytes" refers to cells that can generate eosinophils, monocytes, neutrophils and basophils.

Myelocytes can be obtained during the process of maturing hematopoietic stem cells in the bone marrow to produce leukocytes such as neutrophils, eosinophils, basophils, monocytes, and lymphocytes, erythrocytes, platelets, and the like, which are blood cell components. Myelocytes may be obtained by inducing differentiation from pluripotent stem cells such as iPS cells and ES cells by methods well known to those skilled in the art.

The induction of myelocytes can be confirmed by subjecting the cultured cells to flow cytometry analysis to detect the appearance of cells exhibiting a characteristic cell surface marker expression pattern of myelocytes to be described below or by subjecting the cultured cells to a colony formation assay to confirm that the cells have a differentiation ability of myelocytes.

Myelocytes are a type of blood cells and can be derived from bone marrow cells (hematopoietic stem cells). Hematopoietic stem cells can differentiate into hematopoietic progenitor cells, and hematopoietic progenitor cells can differentiate into blood cells such as erythrocytes, granulocytes, monocytes, macrophages, megakaryocytes and platelets. Examples of granulocytes include myelocytes, promyelocytes, metamyelocytes, myeloblasts, band cells, and segmented cells (neutrophils, eosinophils, basophils, etc.).

In the present embodiment, "progenitor cells of myelocytes" may be granulocyte-macrophage progenitor cells (GMP).

In the present embodiment, "differentiated cells of myelocytes" may be myelocytes, segmented cells (neutrophils, eosinophils, basophils, etc.), monocytes, macrophages and the like.

Myelocytes can be characterized by expressing, for example, the cell surface markers CD11b, CD43, CD16, and CD14, in flow cytometry analysis.

The degree of differentiation of myelocytes can be characterized by the above cell surface marker expression pattern in flow cytometry analysis. For example, when the degree of differentiation of macrophages increases, the expression level of the cell surface markers CD11b and CD14 increases. That is, macrophages having a high degree of differentiation may express the cell surface markers CD11b and CD14. The macrophages having a high degree of differentiation may be CD11b+/CD14+ macrophages in flow cytometry analysis. In addition, when the degree of differentiation of macrophage decreases, the expression level of the cell surface marker CD11b+ decreases. That is, macrophages having a low degree of differentiation may express CD14+, but may not express the cell surface marker CD11b. The macrophages having a low degree of differentiation may be CD11b-/CD14+ macrophages.

### 2. Extraction step

The method for regulating the degree of cell differentiation may further include a step of extracting (isolating or purifying) cells having a desired specific degree of differentiation.

The cells having a specific degree of differentiation may be extracted before or after the MYC family gene and the BMI1 gene are forcibly expressed.

In one aspect, this extraction step may be performed before the MYC family gene and the BMI1 gene are forcibly expressed, and the MYC family gene and the BMI1 gene may be forcibly expressed in the extracted cells having a specific degree of differentiation (for example, cells having a high degree of differentiation).

In another aspect, a cell population containing cells having a desired specific degree of differentiation (for example, cells having a low degree of differentiation) in which the MYC family gene and the BMI1 gene are forcibly expressed is prepared, and the cells having a desired specific degree of differentiation (for example, cells having a low degree of differentiation) are then extracted from the cell population. In the extracted cells having a specific degree of differentiation, the MYC family gene and the BMI1 gene may be forcibly expressed. When target cells are extracted and the method of the present invention is applied to the extracted cells or when target cells are extracted from a cell population to which the method of the present invention is applied and the cells are continuously cultured, the target cell type can be efficiently proliferated. In order to efficiently improve proliferation of the extracted cell type, cells to be extracted are preferably only a single type of cells such as mesenchymal stromal cells, only vascular endothelial cells, or only smooth muscle cells, but a cell population in which two or more types of these cells are mixed may also be used.

Examples of cells to be extracted include mesenchymal stromal cells, vascular endothelial cells, smooth muscle cells, neural crest cells, lymphocytes, megakaryocytes, and myelocytes. Target cells can be extracted using antibodies against cell surface markers specifically expressed (or not expressed) in the cells by methods well known to those skilled in the art such as flow cytometry analysis, panning, and using magnetic beads. Mesenchymal stromal cells, vascular endothelial cells, smooth muscle cells, neural crest cells, lymphocytes, megakaryocytes, and myelocytes can be extracted by isolating cells that exhibits the above cell surface marker expression pattern.

The target cells can be isolated so that the proportion of target cells contained in the cell population after the extraction operation is, for example, 10% or more, 20% or more, 30% or more, 40% or more, 50% or more, 60% or more, 70% or more, 80% or more, 90% or more, or 95% or more (for example, 100%). Single cells of the target cells may be isolated.

### 3. BCL-XL gene

The method for regulating the degree of cell differentiation of the present invention may further include a step of forcibly expressing the BCL-XL gene in mesenchymal stromal cells, vascular endothelial cells, smooth muscle cells, neural crest cells, and lymphocytes having any degree of differentiation, and in megakaryocytes and myelocytes having a high degree of differentiation. When the BCL-XL gene is expressed in addition to the MYC family gene and the BMI1 gene, it can be expected that the regulation of the degree of cell differentiation to a low degree of differentiation will be further promoted.

The period of forced expression of the BCL-XL gene can be appropriately determined by those skilled in the art.

The BCL-XL gene is a gene having a function of inhibiting cell apoptosis.

The MYC family gene, the BMI1 gene, and/or the BCL-XL gene may be forcibly expressed simultaneously or sequentially.

For example, cells of which the degree of cell differentiation is regulated to a low degree of differentiation may be obtained by forcibly expressing the MYC family gene and the BMI1 gene and then forcibly expressing the BCL-XL gene. In addition, cells of which the degree of cell differentiation is regulated to a low degree of differentiation may be obtained by forcibly expressing the MYC family gene, the BMI1 gene, and the BCL-XL gene simultaneously. When the degree of cell differentiation is maintained at a low degree of differentiation, it is preferable to maintain the forced expression of BCL-XL in addition to the MYC family gene and the BMI1 gene during the culture period.

When genes such as the MYC family gene, the BMI1 gene, and the BCL-XL gene are forcibly expressed in cells, it may be performed by any method well known to those skilled in the art. For example, genes may be introduced into cells and expressed using a gene transfer system based on a viral vector such as lentivirus, retrovirus, or Sendai virus or a non-viral vector such as a plasmid vector or an episomal vector. A method in which target genes are non-virally incorporated into the genome of cells using transposons, a stable expression cell line is established, and unnecessary introduced genes are then removed by transposonase (for example, PiggyBac Transposon system) may be used.

Target cells may be transfected with an expression vector (for example, a viral vector) of desired genes (for example, the MYC family gene and the BMI1 gene, optionally additionally the BCL-XL gene), or target cells may be induced from pluripotent stem cells (for example, ES cells and iPS cells) in which an expression cassette of desired genes (for example, the MYC family gene and the BMI1 gene, optionally additionally the BCL-XL gene) is incorporated in advance, and the genes may be forcibly expressed in this stage. Alternatively, in pluripotent stem cells (for example, ES cells and iPS cells) in which an expression cassette of desired genes (for example, the MYC family gene and the BMI1 gene, optionally additionally the BCL-XL gene) is incorporated in advance, the pluripotent stem cells may be induced to differentiate into target cells while the genes are forcibly expressed. When gene expression is performed using a gene transfer vector, the gene may be operably linked downstream of an appropriate promoter, which is then inserted into the gene transfer vector and introduced into cells to express target genes.

The promoter may be an exogenous promoter. In this specification, an "endogenous" promoter of a gene refers to a promoter that is naturally linked to the gene in the genome, and an "exogenous" promoter of a gene refers to one that is artificially positioned in close proximity to the gene by a genetic manipulation (that is, a molecular biological technique) such that transcription of the gene is directed by a promoter to which it is operably linked. Here, "operably" linked means that a promoter and a target gene are linked such that the target gene is regulated in cis by the promoter, and desired expression of the target gene is achieved. The exogenous promoter may be a constitutive promoter or a regulatable promoter. Examples of constitutive promoters include a CMV promoter, an EF1 promoter, and a ubiquitin promoter. The regulatable promoter refers to an inducible or repressible promoter, and refers to a promoter that has a DNA sequence that functions together with a promoter to which either a repressor or an inducer can bind. When a promoter is induced or repressed, the promoter is in "on state" or when a promoter is not induced or not repressed, the promoter is in the "off state." Examples of regulatable promoters include drug-responsive promoters such as a tetracycline-responsive promoter, a steroid-responsive promoter, and a metallothionein promoter. The tetracycline-responsive promoter is a known regulatable promoter that is reversibly controlled in the presence or absence of tetracycline or its derivatives (for example, doxycycline (Dox)). The tetracycline-responsive promoter is a promoter having a tetracycline response element (TRE) disposed therein, and is a promoter that is activated (that is, induces expression of a target protein) when a reverse tetracycline-regulated transactivator (rtTA) protein or a tetracycline-regulated transactivator (tTA) binds to the TRE. The rtTA protein binds to the TRE in the presence of Dox, and on the other hand, the tTA protein binds to the TRE in the absence of Dox, and the expression of a target gene functionally linked to the promoter downstream of the TRE sequence is induced. If a tetracycline-responsive promoter is used, when cells into which the gene functionally linked to the tetracycline-responsive promoter and the rtTA or tTA protein are introduced are cultured in the presence of Dox, the expression of the gene can be induced or inhibited in a Dox dependent manner. The exogenous promoter is preferably a regulatable promoter. When the regulatable promoter is used, target genes can be inducibly expressed by controlling, for example, drug addition. Among such gene expression systems using drugs, those skilled in the art can easily select an appropriate system in order to achieve a desired expression control of the MYC family gene, the BMI1 gene, the BCL-XL gene and the like. In order to achieve such expression, commercially available kits and the like may be used. In addition, expression control target genes, the MYC family gene, the BMI1 gene, and the BCL-XL gene, may be inserted into separate vectors or may be inserted into the same vector.

The MYC family gene, the BMI1 gene, and the BCL-XL gene promote the regulation of the degree of cell differentiation to a low degree of differentiation, but may inhibit terminal differentiation of differentiated cells (for example, adipocytes, osteoblasts, chondrocytes, macrophages, dendritic cells, neutrophils, and erythrocytes) so that the expression of these genes may be inhibited before a terminal differentiation step. When the expression of these genes in cells is inhibited, it becomes easier to induce functional and more mature differentiated cells (for example, adipocytes, osteoblasts, chondrocytes, vascular endothelial cells, vascular smooth muscle cells, megakaryocytes, platelet, macrophages, dendritic cells, neutrophils, erythrocytes, megakaryocytes).

Inhibition of the expression of the MYC family gene, the BMI1 gene, the BCL-XL gene and the like in cells may be achieved, for example, by releasing induction of expression according to a drug-inducible expression system using the above regulatable promoter according to removal of a drug or the like. Alternatively, the introduced MYC family gene, BMI1 gene, BCL-XL gene and the like may be removed using a Cre/lox system or the like, and the expression of these genes may be suppressively controlled. In order to suppresively regulate the expression of the MYC family gene, the BMI1 gene, the BCL-XL gene and the like, commercially available kits and the like can be appropriately used.

For the forced expression of the above genes and its release (inhibition), a commercially available drug-responsive gene expression induction system such as Tet-on (registered trademark) or Tet-off (registered trademark) system may be used. In this case, in the step of forced expression, a corresponding drug, for example, tetracycline or doxycycline, may be added to a medium, and the forced expression may be inhibited by removing the drug from the medium. When a drug (for example, doxycycline)-responsive gene expression induction system is used, it is possible to stably proliferate cells (mesenchymal stromal cells having any degree of differentiation etc.) for a long period in a drug-inducible manner, and by simply removing the drug from the medium at any time, it is possible to inhibit proliferation and simultaneously prepare a large amount of desired differentiated cells. Alternatively, a temperature-sensitive gene expression control system or a light-sensitive gene expression control system may be used.

Forced expression of genes and release (inhibition) of forced expression can be performed by methods described in WO 2011/034073 (mentioned above), U.S. Patent Application Publication No. 2012/0238023, WO 2012/157586 (mentioned above), U.S. Patent Application Publication No. 2014/0127815, WO 2014/123242 and U.S. Patent Application Publication No. 2016/0002599, and Nakamura S et al, Cell Stem Cell. 14, 535-548, 2014, other known methods or methods equivalent thereto. In addition, it can be performed by methods described in WO 2015/046229, WO 2016/125364, and WO 2020/045651, other known methods or methods equivalent thereto.

### 4. CDKN1A gene

The method for regulating the degree of cell differentiation according to the present invention may include a step of inhibiting the expression of at least one of the CDKN1A gene and the p53 gene or the function of expression products thereof in mesenchymal stromal cells, vascular endothelial cells, smooth muscle cells, neural crest cells, and lymphocytes having any degree of differentiation and in megakaryocytes and myelocytes having a high degree of differentiation.

Here, expression is used as a concept including transcription and translation, and inhibiting expression may include inhibiting at a transcription level and inhibiting at a translation level. In the method of the present invention, when the expression of the CDKN1A gene and/or the p53 gene or the function of expression products thereof is inhibited, it can be expected that the regulation of the degree of cell differentiation (regulation to a low degree of differentiation) will be further improved.

The CDKN1A (cyclin-dependent kinase inhibitor 1A) gene encodes a cell cycle inhibitor p21 and is also known as a downstream gene of the tumor suppressor gene p53. The activated p53 protein functions as a transcription factor, and increases the expression level of the p53 downstream gene group. Therefore, as used herein, "inhibiting the expression of genes or the function of expression products thereof" may be achieved by directly inhibiting the expression of target genes or the function of expression products thereof (for example, p21 in the case of the CDKN1A gene), or can also be achieved by controlling the expression of genes upstream of the target genes or the function of expression products thereof. However, in this specification, when the expression of the CDKN1A gene or the function of expression products thereof is inhibited, the upstream gene of the target CDKN1A gene includes neither the p53 gene nor other tumor suppressor genes INK4A and ARF located upstream of the p53 gene.

It is preferable to inhibit the expression of not only the CDKN1A gene but also the p53 gene or the function of expression products thereof.

Inhibition of the expression of the above genes or the function of expression products thereof can be performed by known methods, and for example, it can be achieved by introducing various molecules such as siRNA, shRNA, and antisense nucleic acids (referred to as "expression-inhibiting nucleic acids"), which can specifically inhibit the expression of genes or expression vectors that can express these expression-inhibiting nucleic acids into cells. Alternatively, other techniques such as a genome editing technique may be used to knock down genes. For example, when a gene is knock-down using a CRISPR-Cas system, a guide RNA that targets the gene, and a fusion protein of inactive Cas such as dCas and a repressor domain are used.

Typically, siRNA is a double-stranded oligo RNA composed of RNA having a sequence complementary to a nucleotide sequence or a partial sequence of mRNA of a target gene and its complementary strand. When used in mammalian cells, the length of siRNA is generally about 19 to 30 bases, and preferably 21 to 25 bases. The nucleotide sequence of these RNA can be appropriately designed by those skilled in the art based on sequence information of the gene whose expression is inhibited. In place of siRNA, shRNA can also be used.

The antisense nucleic acid refers to a nucleic acid that has a nucleotide sequence that can specifically hybridize with a target mRNA under physiological conditions of cells that express target mRNA (mature mRNA or initial transcription product) and can inhibit translation of a polypeptide encoded by the target mRNA in the hybridized state. The antisense nucleic acid is generally a single-stranded nucleic acid having a length of 10 to 100 bases, and preferably 15 to 30 bases. The type of the antisense nucleic acid may be DNA or RNA, or a chimera of DNA and RNA. The nucleotide sequence of the antisense nucleic acid can be appropriately designed by those skilled in the art based on sequence information of the gene whose expression is inhibited.

In addition to the above techniques, compounds known to inhibit the expression of genes can also be used. For example, as the compounds that inhibit the expression of the CDKN1A gene, p21 inhibitors such as UC2288, butyrolactone I, LLW10, sorafenib, and sterigmatocystin are known. In addition, pifithrin α, nutlin-3, ReACp53, RG7388 and the like are known as p53 inhibitors.

Alternatively, in order to inhibit the expression of the genes or the function of expression products thereof, target genes may be knockout using known techniques. Knockout of a gene means that the gene is completely or partially destroyed or mutated so that it does not perform its original function. A gene may be destroyed or mutated such that one allelic gene on the genome does not function. In addition, a plurality of allelic genes may be destroyed or mutated. Knockout can be performed by known methods, and examples thereof include a method for knocking out by introducing a DNA construct designed to cause genetic recombination with a target gene into cells and a method for knocking out by introducing base insertion, deletion, or substitution using a genome editing technique such as a TALEN or CRISPR-Cas system.

In addition, compounds that inhibit transcription and transcription products of genes, inhibitors that block binding of the produced protein to target proteins (p53 binding inhibition: pifithrin α, nutlin-3, ReACp53, RG7388, etc.; and p21 binding inhibition: UC2288, butyrolactone I, LLW10, sorafenib, sterigmatocystin, etc.) or the like may be used.

The expression of the genes or the function of expression products thereof can be inhibited by the above methods.

The expression of the CDKN1A gene and/or the p53 gene is preferably inhibited by introducing expression vectors that express expression-inhibiting nucleic acids for the genes into cells. When expression-inhibiting nucleic acids for genes such as the CDKN1A gene and the p53 gene are forcibly expressed in cells, it may be performed by any method well known to those skilled in the art. For example, nucleic acids encoding the expression-inhibiting nucleic acids may be introduced into cells and expressed using a gene transfer system based on a viral vector such as lentivirus or retrovirus or a non-viral vector such as a plasmid vector or an episomal vector. It is also preferable to use a method in which nucleic acids encoding expression-inhibiting nucleic acids are non-virally incorporated into the genome of cells using transposons, stable expression cell lines of the expression-inhibiting nucleic acids are established, and unnecessary introduced nucleic acids are then removed by transposonase (for example, PiggyBac Transposon system). Target cells may be transfected with expression vectors (for example, a viral vector) of expression-inhibiting nucleic acids for desired genes (for example, the CDKN1A gene and the p53 gene), or target cells may be induced from pluripotent stem cells (for example, ES cells and iPS cells) in which an expression cassette of expression-inhibiting nucleic acids for desired genes (for example, the CDKN1A gene and the p53 gene) is incorporated in advance, and the siRNA, shRNA or antisense nucleic acid may be forcibly expressed in this stage. Alternatively, in pluripotent stem cells (for example, ES cells and iPS cells) in which an expression cassette of expression-inhibiting nucleic acids for desired genes (for example, the CDKN1A gene and the p53 gene) is incorporated in advance, the pluripotent stem cells may be induced to differentiate into target cells while the expression-inhibiting nucleic acids are forcibly expressed. When an expression vector is used to express an expression-inhibiting nucleic acid in cells, a nucleic acid (for example, DNA) encoding the expression-inhibiting nucleic acid may be operably linked downstream of an appropriate promoter, which is then inserted into the expression vector and introduced into cells to express a target expression-inhibiting nucleic acid. The promoter may be an exogenous promoter. The exogenous promoter may be a constitutive promoter or a regulatable promoter, and is preferably a constitutive promoter. When relatively small RNA such as siRNA or shRNA is expressed, it is preferable to use pollll promoters such as U6 promoter, H1 promoter, tRNA promoter, retroviral LTR promoter, adenovirus Val promoter, 5S rRNA promoter, 7SK RNA promoter, and 7SL RNA promoter as an example of the constitutive promoter. Nucleic acids encoding expression-inhibiting nucleic acids for the CDKN1A gene and nucleic acids encoding expression-inhibiting nucleic acids for the p53 gene may be inserted into separate expression vectors or may be inserted into the same expression vector.

In the present embodiment, inhibition of the expression of the CDKN1A gene or the p53 gene or the function of expression products thereof may be performed simultaneously with the forced expression of any of the MYC family gene, the BMI1 gene, and the BCL-XL gene, is preferably performed simultaneously with the forced expression of the BCL-XL gene, or can be performed, for example, after a decrease in cell proliferation is confirmed. As an example, inhibition can be performed after a proliferation rate at a certain point is compared with the most recent prior proliferation rate (for example, cell proliferation is confirmed weekly, and a cell proliferation rate in a certain week is compared with a proliferation rate in the previous week), and the proliferation rate that is decreased to one-half or less is confirmed. Although not intended to be limiting, the decrease in cell proliferation can be observed from immediately after the forced expression of the MYC family gene and the BMI1 gene up to about 30 days, about 40 days, about 50 days, about 60 days, about 70 days, about 80 days, or about 90 days. In one aspect, in target cells, the MYC family gene (for example, the c-Myc gene) and the BMI1 gene are forcibly expressed, and concurrently, the expression of the CDKN1A gene and/or the p53 gene or the function of expression products thereof is inhibited. In one aspect, in target cells, the MYC family gene (for example, the c-Myc gene), the BMI1 gene, and the BCL-XL gene are forcibly expressed, and concurrently, the expression of the CDKN1A gene and/or the p53 gene or the function of expression products thereof is inhibited.

### 5. Homolog

As used herein, genes such as the MYC family gene, the BMI1 gene, the BCL-XL gene, the CDKN1A gene, and the p53 gene refer to those encoded by their known nucleic acid sequences, for example, cDNA sequences. The genes may also include homologs that are identified based on the homology of known nucleic acid sequences. A "homolog" is a gene whose cDNA sequence is substantially identical to the nucleic acid sequence of the gene.

Among MYC family genes, a homolog of the c-MYC gene is a gene whose cDNA sequence is substantially identical to, for example, the nucleic acid sequence shown in SEQ ID NO. 1. cDNA having a sequence substantially identical to the nucleic acid sequence shown in SEQ ID NO. 1 is DNA having a sequence having an identity of about 60% or more, preferably about 70% or more, more preferably about 80% or more, for example, 81%, 82%, 83%, 84%, 85%, 86%, 87%, 88%, or 89%, still more preferably about 90% or more, for example, 91%, 92%, 93%, 94%, 95%, 96%, 97%, or 98%, and most preferably about 99% or more to the DNA having the sequence shown in SEQ ID NO. 1, or DNA that can be hybridized under stringency conditions with DNA or RNA having a sequence complementary to the nucleic acid sequence shown in SEQ ID NO. 1 and the proteins encoded by these DNA inhibit the cell cycle. Alternatively, cDNA having a sequence substantially identical to the nucleic acid sequence shown in SEQ ID NO. 1 is DNA having a sequence in which one or more bases, for example, 1 to 10 bases, preferably several bases, for example, 1 to 5 bases, 1 to 4 bases, 1 to 3 bases, or 1 to 2 bases in the sequence shown in SEQ ID NO. 1 are deleted, substituted or added, and the proteins encoded by these DNA inhibit the cell cycle. The c-MYC gene may be a gene encoding c-MYC fused to a destabilization domain (DD). The destabilization domain purchased from ProteoTuner or Clontech can be used.

The sequence shown in SEQ ID NO. 1 is the following sequence.

atgcccctca acgttagctt caccaacagg aactatgacc tcgactacga ctcggtgcag ccgtatttct

actgcgacga ggaggagaac ttctaccagc agcagcagca gagcgagctg cagcccccgg

cgcccagcga ggatatctgg aagaaattcg agctgctgcc caccccgccc ctgtccccta gccgccgctc

cgggctctgc tcgccctcct acgttgcggt cacacccttc tcccttcggg gagacaacga cggcggtggc

gggagcttct ccacggccga ccagctggag atggtgaccg agctgctggg aggagacatg gtgaaccaga

gtttcatctg cgacccggac gacgagacct tcatcaaaaa catcatcatc caggactgta tgtggagcgg

cttctcggcc gccgccaagc tcgtctcaga gaagctggcc tcctaccagg ctgcgcgcaa agacagcggc

agcccgaacc ccgcccgcgg ccacagcgtc tgctccacct ccagcttgta cctgcaggat ctgagcgccg

ccgctcagag tgcatcgacc cctcggtggt cttcccctac cctctcaacg acagcagctc gcccaagtcc

tgcgcctcgc aagactccag cgccttctct ccgtcctcgg attctctgct ctcctcgacg gagtcctccc

cgcagggcag ccccgagccc ctggtgctcc atgaggagac accgcccacc accagcagcg actctgagga

ggaacaagaa gatgaggaag aaatcgatgt tgtttctgtg gaaaagaggc aggctcctgg caaaaggtca

gagtctggat caccttctgc tggaggccac agcaaacctc ctcacagccc actggtcctc aagaggtgcc

acgtctccac acatcagcac aactacgcag cgcctccctc cactcggaag gactatcctg ctgccaagag

ggtcaagttg gacagtgtca gagtcctgag acagatcagc aacaaccgaa aatgcaccag ccccaggtcc

tcggacaccg aggagaatgt caagaggcga acacacaacg tcttggagcg ccagaggagg

aacgagctaa aacggagctt ttttgccctg cgtgaccaga tcccggagtt ggaaaacaat gaaaaggccc

ccaaggtagt tatccttaaa aaagccacag catacatcct gtccgtccaa gcagaggagc aaaagctcat

ttctgaagag gacttgttgc ggaaacgacg agaacagttg aaacacaaac ttgaacagct acggaactct

tgtgcgtaa

A homolog of the BMI1 gene is a gene whose cDNA sequence is substantially identical to, for example, the nucleic acid sequence shown in SEQ ID NO. 2. cDNA having a sequence substantially identical to the nucleic acid sequence shown in SEQ ID NO. 2 is DNA having a sequence having an identity of about 60% or more, preferably about 70% or more, more preferably about 80% or more, for example, 81%, 82%, 83%, 84%, 85%, 86%, 87%, 88%, or 89%, still more preferably about 90% or more, for example, 91%, 92%, 93%, 94%, 95%, 96%, 97%, or 98%, and most preferably about 99% or more to the DNA having the sequence shown in SEQ ID NO. 2 or DNA that can be hybridized under stringency conditions with DNA or RNA having a sequence complementary to the nucleic acid sequence shown in SEQ ID NO. 2, and the proteins encoded by these DNA inhibit the cell cycle. Alternatively, cDNA having a sequence substantially identical to the nucleic acid sequence shown in SEQ ID NO. 2 is DNA having a sequence in which one or more bases, for example, 1 to 10 bases, preferably several bases, for example, 1 to 5 bases, 1 to 4 bases, 1 to 3 bases, or 1 to 2 bases in the sequence shown in SEQ ID NO. 2 are deleted, substituted or added, and the proteins encoded by these DNA inhibit the cell cycle.

The sequence shown in SEQ ID NO. 2 is the following sequence.

atgcatcgaa caacgagaat caagatcact gagctaaatc cccacctgat gtgtgtgctt tgtggagggt

acttcattga tgccacaacc ataatagaat gtctacattc cttctgtaaa acgtgtattg ttcgttacct

ggagaccagc aagtattgtc ctatttgtga tgtccaagtt cacaagacca gaccactact gaatataagg

tcagataaaa ctctccaaga tattgtatac aaattagttc cagggctttt caaaaatgaa atgaagagaa

gaagggattt ttatgcagct catccttctg ctgatgctgc caatggctct aatgaagata gaggagaggt

tgcagatgaa gataagagaa ttataactga tgatgagata ataagcttat ccattgaatt ctttgaccag

aacagattgg atcggaaagt aaacaaagac aaagagaaat ctaaggagga ggtgaatgat aaaagatact

tacgatgccc agcagcaatg actgtgatgc acttaagaaa gtttctcaga agtaaaatgg acatacctaa

tactttccag attgatgtca tgtatgagga ggaaccttta aaggattatt atacactaat ggatattgcc

tacatttata cctggagaag gaatggtcca cttccattga aatacagagt tcgacctact tgtaaaagaa

tgaagatcag tcaccagaga gatggactga caaatgctgg agaactggaa agtgactctg ggagtgacaa

ggccaacagc ccagcaggag gtattccctc cacctcttct tgtttgccta gccccagtac tccagtgcag

tctcctcatc cacagtttcc tcacatttcc agtactatga atggaaccag caacagcccc agcggtaacc

accaatcttc ttttgccaat agacctcgaa aatcatcagt aaatgggtca tcagcaactt cttctggttg a

A homolog of the BCL-XL gene is a gene whose cDNA sequence is substantially identical to, for example, the nucleic acid sequence shown in SEQ ID NO. 3. cDNA having a sequence substantially identical to the nucleic acid sequence shown in SEQ ID NO. 3 is DNA having a sequence having an identity of about 60% or more, preferably about 70% or more, more preferably about 80% or more, for example, 81%, 82%, 83%, 84%, 85%, 86%, 87%, 88%, or 89%, still more preferably about 90% or more, for example, 91%, 92%, 93%, 94%, 95%, 96%, 97%, or 98%, and most preferably about 99% or more to the DNA having the sequence shown in SEQ ID NO. 3 or DNA that can be hybridized under stringency conditions with DNA or RNA having a sequence complementary to the nucleic acid sequence shown in SEQ ID NO. 3, and the proteins encoded by these DNA inhibit the cell cycle. Alternatively, cDNA having a sequence substantially identical to the nucleic acid sequence shown in SEQ ID NO. 3 is DNA having a sequence in which one or more bases, for example, 1 to 10 bases, preferably several bases, for example, 1 to 5 bases, 1 to 4 bases, 1 to 3 bases, or 1 to 2 bases in the sequence shown in SEQ ID NO. 3 are deleted, substituted or added, and the proteins encoded by these DNA inhibit the cell cycle.

The sequence shown in SEQ ID NO. 3 is the following sequence.

atgtctcaga gcaaccggga gctggtggtt gactttctct cctacaagct ttcccagaaa ggatacagct

ggagtcagtt tagtgatgtg gaagagaaca ggactgaggc cccagaaggg actgaatcgg agatggagac

ccccagtgcc atcaatggca acccatcctg gcacctggca gacagccccg cggtgaatgg agccactggc

cacagcagca gtttggatgc ccgggaggtg atccccatgg cagcagtaaa gcaagcgctg agggaggcag

gcgacgagtt tgaactgcgg taccggcggg cattcagtga cctgacatcc cagctccaca tcaccccagg

gacagcatat cagagctttg aacaggtagt gaatgaactc ttccgggatg gggtaaactg gggtcgcatt

gtggcctttt tctccttcgg cggggcactg tgcgtggaaa gcgtagacaa ggagatgcag gtattggtga

gtcggatcgc agcttggatg gccacttacc tgaatgacca cctagagcct tggatccagg agaacggcgg

ctgggatact tttgtggaac tctatgggaa caatgcagca gccgagagcc gaaagggcca ggaacgcttc

aaccgctggt tcctgacggg catgactgtg gccggcgtgg ttctgctggg ctcactcttc agtcggaaat ga

A homolog of the CDKN1A gene is a gene whose cDNA sequence is substantially identical to, for example, the nucleic acid sequence shown in SEQ ID NO. 4. cDNA having a sequence substantially identical to the nucleic acid sequence shown in SEQ ID NO. 4 is DNA having a sequence having an identity of about 60% or more, preferably about 70% or more, more preferably about 80% or more, for example, 81%, 82%, 83%, 84%, 85%, 86%, 87%, 88%, or 89%, still more preferably about 90% or more, for example, 91%, 92%, 93%, 94%, 95%, 96%, 97%, or 98%, and most preferably about 99% or more to the DNA having the sequence shown in SEQ ID NO. 4 or DNA that can be hybridized under stringency conditions with DNA or RNA having a sequence complementary to the nucleic acid sequence shown in SEQ ID NO. 4, and the proteins encoded by these DNA inhibit the cell cycle. Alternatively, cDNA having a sequence substantially identical to the nucleic acid sequence shown in SEQ ID NO. 4 is DNA having a sequence in which one or more bases, for example, 1 to 10 bases, preferably several bases, for example, 1 to 5 bases, 1 to 4 bases, 1 to 3 bases, or 1 to 2 bases in the sequence shown in SEQ ID NO. 4 are deleted, substituted or added, and the proteins encoded by these DNA inhibit the cell cycle.

The sequence shown in SEQ ID NO. 4 is the following sequence.

atgtggggag tattcaggag acagacaact cactcgtcaa atcctcccct tcctggccaa caaagctgct
gcaaccacag ggatttcttc tgttcaggcg ccatgtcaga accggctggg gatgtccgtc agaacccatg
cggcagcaag gcctgccgcc gcctcttcgg cccagtggac agcgagcagc tgagccgcga ctgtgatgcg
ctaatggcgg gctgcatcca ggaggcccgt gagcgatgga acttcgactt tgtcaccgag acaccactgg
agggtgactt cgcctgggag cgtgtgcggg gccttggcct gcccaagctc taccttccca cggggccccg
gcgaggccgg gatgagttgg gaggaggcag gcggcctggc acctcacctg ctctgctgca ggggacagca
gaggaagacc atgtggacct gtcactgtct tgtacccttg tgcctcgctc aggggagcag gctgaagggt
ccccaggtgg acctggagac tctcagggtc gaaaacggcg gcagaccagc atgacagatt tctaccactc
caaacgccgg ctgatcttct ccaagaggaa gccctaa

The p53 gene is a gene whose cDNA sequence is substantially identical to, for example, the nucleic acid sequence shown in SEQ ID NO. 5. cDNA having a sequence substantially identical to the nucleic acid sequence shown in SEQ ID NO. 5 is DNA having a sequence having an identity of about 60% or more, preferably about 70% or more, more preferably about 80% or more, for example, 81%, 82%, 83%, 84%, 85%, 86%, 87%, 88%, or 89%, still more preferably 90% or more, for example, 91%, 92%, 93%, 94%, 95%, 96%, 97%, or 98%, and most preferably about 99% or more to DNA having the sequence shown in SEQ ID NO. 5 or DNA that can be hybridized under stringency conditions with DNA having a sequence complementary to the nucleic acid sequence shown in SEQ ID NO. 5, and the proteins encoded by the DNA inhibit cancer. Alternatively, cDNA having a sequence substantially identical to the nucleic acid sequence shown in SEQ ID NO. 5 is DNA having a sequence in which one or more bases, for example, 1 to 10 bases, preferably several bases, for example, 1 to 5 bases, 1 to 4 bases, 1 to 3 bases, or 1 to 2 bases in the sequence shown in SEQ ID NO. 5 are deleted, substituted or added, and the proteins encoded by these DNA inhibit cancer.

The sequence shown in SEQ ID NO. 5 is the following sequence.

atggaggagc cgcagtcaga tcctagcgtc gagccccctc tgagtcagga aacattttca gacctatgga
aactacttcc tgaaaacaac gttctgtccc ccttgccgtc ccaagcaatg gatgatttga tgctgtcccc
ggacgatatt gaacaatggt tcactgaaga cccaggtcca gatgaagctc ccagaatgcc agaggctgct
ccccccgtgg cccctgcacc agcagctcct acaccggcgg cccctgcacc agccccctcc tggcccctgt
catcttctgt cccttcccag aaaacctacc agggcagcta cggtttccgt ctgggcttct tgcattctgg
gacagccaag tctgtgactt gcacgtactc ccctgccctc aacaagatgt tttgccaact ggccaagacc
tgccctgtgc agctgtgggt tgattccaca cccccgcccg gcacccgcgt ccgcgccatg gccatctaca
agcagtcaca gcacatgacg gaggttgtga ggcgctgccc ccaccatgag cgctgctcag atagcgatgg
tctggcccct cctcagcatc ttatccgagt ggaaggaaat ttgcgtgtgg agtatttgga tgacagaaac
acttttcgac atagtgtggt ggtgccctat gagccgcctg aggttggctc tgactgtacc accatccact
acaactacat gtgtaacagt tcctgcatgg gcggcatgaa ccggaggccc atcctcacca tcatcacact

ggaagactcc agtggtaatc tactgggacg gaacagcttt gaggtgcgtg tttgtgcctg tcctgggaga

gaccggcgca cagaggaaga gaatctccgc aagaaagggg agcctcacca cgagctgccc

ccagggagca ctaagcgagc actgcccaac aacaccagct cctctcccca gccaaagaag aaaccactgg

atggagaata tttcaccctt cagatccgtg ggcgtgagcg cttcgagatg ttccgagagc tgaatgaggc

cttggaactc aaggatgccc aggctgggaa ggagccaggg gggagcaggg ctcactccag ccacctgaag

tccaaaaagg gtcagtctac ctcccgccat aaaaaactca tgttcaagac agaagggcct gactcagact ga

Here, stringency conditions refer to hybridization conditions that can be easily determined by those skilled in the art, and are generally empirical experiment conditions that depend on the base length of the nucleic acid, the washing temperature, and the salt concentration. Generally, when the length of the base is long, the temperature for appropriate annealing increases, and when the length of the base is short, the temperature decreases. Hybridization generally depends on the ability of complementary strands to reanneal in an environment slightly below melting points thereof.

Specifically, for example, low stringency conditions include washing in a 0.1×SSC and 0.1% SDS solution under a temperature condition of 37°C to 42°C in the washing stage of the filter after hybridization. In addition, examples of high stringency conditions include washing at 65°C, 5×SSC and 0.1% SDS in the washing stage. By increasing the stringency of conditions, polynucleotides with higher homology can be obtained.

### 6. Culturing step

A method for producing progenitor cells of mesenchymal stromal cells, vascular endothelial cells, smooth muscle cells, neural crest cells, lymphocytes, megakaryocytes, or myelocytes according to the present embodiment includes a step of culturing cells obtained by the method for regulating the degree of cell differentiation according to the present embodiment (culturing step).

The term "progenitor cells" refers to cells in a process of differentiating from cells having a proliferative ability into differentiated cells which are in the final differentiated form.

Cell culture conditions can be appropriately determined by those skilled in the art depending on the type and state of cells. For example, the culture temperature can be about 35°C to about 42°C, about 36°C to about 40°C, or about 37°C to about 39°C, the carbon dioxide concentration can be, for example, 5% CO₂, and the oxygen concentration can be, for example, 20% O₂. The culture may be stationary culture or shaking culture. The shaking speed in the case of shaking culture is not particularly limited, and can be, for example, 10 rpm to 200 rpm, or 30 rpm to 150 rpm.

The medium may be an Iscove's modified Dulbecco's medium (IMDM) containing serum, insulin, transferrin, serine, thioglycerol, ascorbic acid, and TPO. In this case, the IMDM medium may further contain SCF and may further contain heparin. In addition, phorbol esters (for example, phorbol-12-myristate-13-acetate; PMA) may be added.

The step of culturing cells can be performed in the presence or absence of feeder cells. In this specification, the term "feeder cells" refers to cells that are co-cultured with target cells in order to provide an environment necessary for culture of the target cells to be proliferated or differentiated. Feeder cells may be cells of the same species or different species as long as they are cells that can be distinguished from target cells. Feeder cells may be cells that are treated with antibiotic substances or gamma rays in order to prevent proliferation or may be untreated cells.

The medium may contain serum or plasma or may be serum-free. When serum is used, human serum is preferable. As necessary, for example, the medium may also contain one or more substances such as albumin, insulin, transferrin, selenium, fatty acids, trace elements, 2-mercaptoethanol, thioglycerol, monothioglycerol (MTG), lipids, amino acids (for example, L-glutamine), ascorbic acid, heparin, non-essential amino acids, vitamins, growth factors, low-molecular-weight compounds, antibiotic substance, antioxidants, pyruvate, buffer agents, inorganic salts, and cytokines. Examples of cytokines include vascular endothelial growth factors (VEGF), thrombopoietin (TPO), various TPO-like substances, stem cell factors (SCF), erythropoietin (EPO), granulocyte colony-stimulating factors (G-CSF), interleukin 3(IL3), ITS (insulin-transferrin-selenite) supplements, and ADAM (A Disintegrin And Metalloprotease) inhibitors.

### 7. Differentiation step

A method for producing differentiated cells of mesenchymal stromal cells, vascular endothelial cells, smooth muscle cells, neural crest cells, lymphocytes, megakaryocytes, or myelocytes according to the present embodiment includes a step of differentiating cells obtained by the above method for regulating the degree of cell differentiation according to the present embodiment (differentiation step).

The term "differentiated cells" refers to cells in a process of differentiating from cells having a proliferative ability or cells in the final differentiated form.

The differentiation step can be performed by culturing under culture conditions according to the type of cells to be differentiated, and the description of the above culturing step may be referred to.

### 8. Cells

The present invention provides mesenchymal stromal cells, vascular endothelial cells, smooth muscle cells, neural crest cells, lymphocytes, megakaryocytes, or myelocytes, or progenitor cells or differentiated cells thereof, which include the MYC family gene operably linked to the first exogenous promoter and the BMI1 gene operably linked to the second exogenous promoter (hereinafter referred to as cells of the present invention).

The MYC family gene is preferably c-MYC. The first exogenous promoter and the second exogenous promoter may independently be a constitutive promoter or a regulatable promoter, and are preferably a regulatable promoter. The regulatable promoter is preferably a drug-responsive promoter, and more preferably a tetracycline-responsive promoter. The first exogenous promoter and the second exogenous promoter may be of the same type or different types, but the promoters of the same type are preferable. When the promoters of the same type are used, the MYC family gene and the BMI1 gene can be expressed synchronously and the expression can be inhibited synchronously. The first exogenous promoter and the second exogenous promoter are preferably the same regulatable promoter (for example, a drug-responsive promoter), and more preferably both are a tetracycline-responsive promoter. The first exogenous promoter and the second exogenous promoter may independently be operably linked to the MYC family gene and the BMI1 gene, or the MYC family gene and the BMI1 gene may be operably linked to one exogenous promoter. In this case, the MYC family gene and the BMI1 gene are linked via an intervening sequence such as IRES, and bicistronic expression is possible under control of one exogenous promoter. The MYC family gene operably linked to the first exogenous promoter and the BMI1 gene operably linked to the second exogenous promoter may be incorporated into the genome of the cells of the present invention or may be present in an expression vector introduced into the cells of the present invention. Preferably, the MYC family gene operably linked to the first exogenous promoter and the BMI1 gene operably linked to the second exogenous promoter are incorporated into the genome of the cells of the present invention.

When a tetracycline-responsive promoter is used as the first exogenous promoter and/or second exogenous promoter, since tetracycline-dependent expression control is possible, the cells of the present invention preferably further include the rtTA gene or the tTA gene operably linked to the third exogenous promoter. The third exogenous promoter may be a constitutive promoter or a regulatable promoter, and is preferably a constitutive promoter. The rtTA gene or the tTA gene operably linked to the third exogenous promoter may be incorporated into the genome of the cells of the present invention or may be present in an expression vector introduced into the cells of the present invention. Preferably, the rtTA gene or the tTA gene operably linked to the third exogenous promoter may be incorporated into the genome of the cells of the present invention.

When cultured under conditions in which mesenchymal stromal cells, vascular endothelial cells, smooth muscle cells, neural crest cells, lymphocytes, megakaryocytes, or myelocytes can proliferate, the cells of the present invention express the MYC family gene (for example, the c-Myc gene) and the BMI1 gene in an amount capable of promoting the proliferation of these cells in vitro. The amount of the MYC family gene (for example, c-Myc) and the BMI1 gene capable of promoting the proliferation of these cells in vitro is the amount of the MYC family gene and the BMI1 gene such that the proliferation rate of these cells expressing that amount of the MYC family gene and the BMI1 gene is significantly increased compare to mesenchymal stromal cells, vascular endothelial cells, smooth muscle cells, neural crest cells, lymphocytes, megakaryocytes, or myelocytes prepared in the same manner as these cells except that they do not express the MYC family gene and the BMI1 gene.

In order to enhance the proliferative ability, the cells of the present invention may further include the BCL-XL gene operably linked to the fourth exogenous promoter. When the cells are cultured under conditions in which the fourth exogenous promoter operates, it can be expected that the BCL-XL gene will be expressed, and the proliferation of the cells of the present invention will be further promoted. The fourth exogenous promoter may independently be a constitutive promoter or a regulatable promoter, and is preferably a regulatable promoter. The regulatable promoter is preferably a drug-responsive promoter, and more preferably a tetracycline-responsive promoter. The type of the fourth exogenous promoter may be the same as or different from the type of the first exogenous promoter and/or the second exogenous promoter, and preferably, the first, second and fourth exogenous promoters are promoters of the same type. When the same type of promoter is used, the MYC family gene, the BMI1 gene and the BCL-XL gene can be expressed synchronously and the expression can be inhibited synchronously. The first, second and fourth exogenous promoters are preferably the same regulatable promoter (for example, a drug-responsive promoter), and more preferably all are a tetracycline-responsive promoter. The fourth exogenous promoter may be operably linked to the BCL-XL gene independently of the first and second exogenous promoters, the MYC family gene and the BCL-XL gene may be operably linked to the first exogenous promoter, the BMI1 gene and the BCL-XL gene may be operably linked to the second exogenous promoter, and the MYC family gene, the BMI1 gene and the BCL-XL gene may be operably linked to one exogenous promoter. When a plurality of genes are linked via an intervening sequence such as RES, bicistronic expression is possible under control of one exogenous promoter. The BCL-XL gene operably linked to the fourth exogenous promoter may be incorporated into the genome of the cells of the present invention or may be present in an expression vector introduced into the cells of the present invention. Preferably, the BCL-XL gene operably linked to the fourth exogenous promoter is incorporated into the genome of the cells of the present invention.

In order to enhance the proliferative ability, the cells of the present invention may further include nucleic acids encoding expression-inhibiting nucleic acids (for example, siRNA, shRNA, and antisense nucleic acids) for the CDKN1A gene operably linked to the fifth exogenous promoter and/or nucleic acids encoding expression-inhibiting nucleic acids for the p53 gene operably linked to the sixth exogenous promoter. When the cells are cultured under conditions in which the fifth exogenous promoter and/or the sixth exogenous promoter operates, it can be expected that the expression-inhibiting nucleic acids for the CDKN1A gene and/or the expression-inhibiting nucleic acids for the p53 gene will be expressed, and the proliferation of the cells of the present invention will be further promoted. The fifth exogenous promoter and the sixth exogenous promoter may independently be a constitutive promoter or a regulatable promoter, and are preferably a constitutive promoter. The constitutive promoter is preferably a pollll promoter such as H1 promoter. The type of the fifth exogenous promoter may be the same as or different from the type of the sixth exogenous promoter. The nucleic acids encoding expression-inhibiting nucleic acids for the CDKN1A gene operably linked to the fifth exogenous promoter, and the nucleic acids encoding expression-inhibiting nucleic acids for the p53 gene operably linked to the sixth exogenous promoter may be incorporated into the genome of the cells of the present invention or may be present in an expression vector introduced into the cells of the present invention, and preferably, the nucleic acids are incorporated into the genome of the cells of the present invention.

In one aspect, the cells of the present invention are mesenchymal stromal cells, vascular endothelial cells, smooth muscle cells, neural crest cells, lymphocytes, megakaryocytes, or myelocytes, or progenitor cells thereof, which include the MYC family gene operably linked to the first exogenous promoter, the BMI1 gene operably linked to the second exogenous promoter, and the BCL-XL gene operably linked to the fourth exogenous promoter. When a tetracycline-responsive promoter is used as the first exogenous promoter and/or second exogenous promoter, the cells of the present invention may further include the rtTA gene or the tTA gene operably linked to the third exogenous promoter.

In one aspect, the cells of the present invention are mesenchymal stromal cells, vascular endothelial cells, smooth muscle cells, neural crest cells, lymphocytes, megakaryocytes, or myelocytes, or progenitor cells thereof, which include the MYC family gene operably linked to the first exogenous promoter, the BMI1 gene operably linked to the second exogenous promoter, nucleic acids encoding expression-inhibiting nucleic acids for the CDKN1A gene operably linked to the fifth exogenous promoter, and nucleic acids encoding expression-inhibiting nucleic acids for the p53 gene operably linked to the sixth exogenous promoter. When a tetracycline-responsive promoter is used as the first exogenous promoter and/or second exogenous promoter, the cells of the present invention may further include the rtTA gene or the tTA gene operably linked to the third exogenous promoter.

In one aspect, the cells of the present invention are mesenchymal stromal cells, vascular endothelial cells, smooth muscle cells, neural crest cells, lymphocytes, megakaryocytes, or myelocytes, or progenitor cells thereof, which include the MYC family gene operably linked to the first exogenous promoter, the BMI1 gene operably linked to the second exogenous promoter, the BCL-XL gene operably linked to the fourth exogenous promoter, nucleic acids encoding expression-inhibiting nucleic acids for the CDKN1A gene operably linked to the fifth exogenous promoter, and nucleic acids encoding expression-inhibiting nucleic acids for the p53 gene operably linked to the sixth exogenous promoter. When a tetracycline-responsive promoter is used as the first exogenous promoter and/or second exogenous promoter, the cells of the present invention may further include the rtTA gene or the tTA gene operably linked to the third exogenous promoter.

The cells of the present invention can be obtained by the above method of the present invention for regulating the degree of differentiation of mesenchymal stromal cells, vascular endothelial cells, smooth muscle cells, neural crest cells, or lymphocytes having any degree of differentiation, or megakaryocytes or myelocytes having a high degree of differentiation or the above method of the present invention for producing progenitor cells of mesenchymal stromal cells, vascular endothelial cells, smooth muscle cells, neural crest cells, lymphocytes, megakaryocytes, or myelocytes.

In addition, the present invention provides a cell population containing the cells of the present invention (referred to as a cell population of the present invention). The cell population abundantly contains the cells of the present invention, and the proportion of the cells of the present invention included in the entire cell population is, for example, 10% or more, 20% or more, 30% or more, 40% or more, 50% or more, 60% or more, 70% or more, 80% or more, 90% or more, or 95% or more (for example, 100%). Such a cell population abundantly containing the cells of the present invention can be obtained by extracting cells (mesenchymal stromal cells, vascular endothelial cells, smooth muscle cells, neural crest cells, lymphocytes, megakaryocytes, or myelocytes, or progenitor cells thereof) having a desired specific degree of differentiation (for example, a high degree of differentiation and a low degree of differentiation) from the cell population to which the method of the present invention is applied. In a preferable aspect, the cell population of the present invention abundantly containing the cells of the present invention having a specific degree of differentiation (for example, a high degree of differentiation and a low degree of differentiation). In one aspect, the proportion of the cells of the present invention (the cells are mesenchymal stromal cells) included in the entire cell population is 10% or more, 20% or more, 30% or more, 40% or more, 50% or more, 60% or more, 70% or more, 80% or more, 90% or more, or 95% or more (for example, 100%). In one aspect, the proportion of the cells of the present invention (the cells are vascular endothelial cells) included in the entire cell population is 10% or more, 20% or more, 30% or more, 40% or more, 50% or more, 60% or more, 70% or more, 80% or more, 90% or more, or 95% or more (for example, 100%). In one aspect, the proportion of the cells of the present invention (the cells are smooth muscle cells) included in the entire cell population is 10% or more, 20% or more, 30% or more, 40% or more, 50% or more, 60% or more, 70% or more, 80% or more, 90% or more, or 95% or more (for example, 100%). In one aspect, the proportion of the cells of the present invention (the cells are neural crest cells) included in the entire cell population is 10% or more, 20% or more, 30% or more, 40% or more, 50% or more, 60% or more, 70% or more, 80% or more, 90% or more, or 95% or more (for example, 100%). In one aspect, the proportion of the cells of the present invention (the cells are lymphocytes) included in the entire cell population is 10% or more, 20% or more, 30% or more, 40% or more, 50% or more, 60% or more, 70% or more, 80% or more, 90% or more, or 95% or more (for example, 100%). In one aspect, the proportion of the cells of the present invention (the cells are megakaryocytes) included in the entire cell population is 10% or more, 20% or more, 30% or more, 40% or more, 50% or more, 60% or more, 70% or more, 80% or more, 90% or more, or 95% or more (for example, 100%). In one aspect, the proportion of the cells of the present invention (the cells are myelocytes) included in the entire cell population is 10% or more, 20% or more, 30% or more, 40% or more, 50% or more, 60% or more, 70% or more, 80% or more, 90% or more, or 95% or more (for example, 100%). In this manner, a cell population abundantly containing the cells at a specific differentiation stage (for example, a high degree of differentiation and a low degree of differentiation) of the present invention can be obtained from the cell population to which the method of the present invention is applied by isolating and extracting cells at a desired differentiation stage using a cell sorter or the like using antibodies against cell surface markers that are specifically expressed in cells at the differentiation stage.

The cells of the present invention and the cell population of the present invention can be obtained by the above method of the present invention for regulating the degree of differentiation of mesenchymal stromal cells, vascular endothelial cells, smooth muscle cells, neural crest cells, or lymphocytes having any degree of differentiation, or megakaryocytes or myelocytes having a high degree of differentiation or the above method of the present invention for producing progenitor cells of mesenchymal stromal cells, vascular endothelial cells, smooth muscle cells, neural crest cells, lymphocytes, megakaryocytes, or myelocytes.

### 9. Cell preparation

In addition, the present invention provides a cell preparation containing the cell population of the present invention (referred to as a cell preparation of the present invention). The cell preparation of the present invention can be prepared by suspending the cell population of the present invention in an appropriate physiological aqueous solution (for example, saline, an isotonic solution containing glucose and other adjunct agents, and a liquid medium). The physiological aqueous solution may contain a buffer agent (for example, a phosphate buffer and a sodium acetate buffer), a soothing agent (for example, lidocaine hydrochloride, procaine hydrochloride, etc.), a stabilizing agent (for example, human serum albumin, polyethylene glycol, etc.), a preservative (for example, sodium benzoate, benzalkonium chloride, etc.), an antioxidant (for example, ascorbic acid, sodium edetate, etc.) and the like. In the cell preparation, the cell population of the present invention is suspended so that the cell concentration is, for example, 1.0×10¹ to 1.0×10¹² cells/mL.

Depending on the type of the cells or the cell population of the present invention contained in the cell preparation, a combination of cytokines suitable for proliferation of the cells may be added to the cell preparation. For example, in the case of a cell preparation containing mesenchymal stromal cells, bFGF can be added to the cell preparation. In the case of a cell preparation containing vascular endothelial cells, VEGF can be added to the cell preparation. In the case of a cell preparation containing smooth muscle cells, VEGF can be added to the cell preparation. In the case of a cell preparation containing neural crest cells, an ALK5 inhibitor such as SB-431542 and a GSK3 inhibitor such as CHIR99021 can be added to the cell preparation. Alternatively, in the case of a cell preparation containing neural crest cells, an ALK5 inhibitor such as SB-431542, and EGF and FGF2 may be added to the cell preparation. In the case of a cell preparation containing lymphocytes, IL-3 can be added to the cell preparation. In the case of a cell preparation containing megakaryocytes, TPO and SCF can be added to the cell preparation.

### 10. Agent for regulating degree of cell differentiation

An agent for regulating the degree of cell differentiation according to the present embodiment, which contains molecules that forcibly express the MYC family gene and the BMI1 gene as an active component, optionally contains molecules that forcibly express the BCL-XL gene as an active component or contains molecules that inhibit the expression of the CDKN1A gene or the p53 gene or the function of expression products thereof as an active component.

### 11. Pharmaceutical composition

A pharmaceutical composition according to the present embodiment contains cells obtained by the method for regulating the degree of cell differentiation of the present invention or the method for producing progenitor cells or differentiated cells of mesenchymal stromal cells, vascular endothelial cells, smooth muscle cells, neural crest cells, lymphocytes, megakaryocytes, or myelocytes of the present invention.

### 12. Treatment or prevention method

A treatment or prevention method according to the present embodiment includes administering cells obtained by the method for regulating the degree of cell differentiation of the present invention or the method for producing progenitor cells or differentiated cells of mesenchymal stromal cells, vascular endothelial cells, smooth muscle cells, neural crest cells, lymphocytes, megakaryocytes, or myelocytes of the present invention, or the pharmaceutical composition containing the cells of the present invention to a subject in need thereof.

Hereinafter, the present invention will be described in detail with reference to examples, but the present invention is not limited thereto. Those skilled in the art can modify the present invention in various aspects without departing from the spirit of the present invention, and such modifications are also included in the scope of the present invention.

### Examples

### Example 1-1: Mesenchymal stromal cells

According to the report of Fukuta et al. (PlosOne, 2014, 9(12): e112291), neural crest cells (NCCs) were induced from iPS cells in a Matrigel-coated culture dish in the presence of SB-431542, and CHIR99021 after 7 days of culture. Then, the medium was changed to a medium containing EGF, FGF2, and SB-431542 in a fibronectin-coated culture dish, and the NCCs were amplified. In addition, the medium was changed to a medium containing 10% FBS and FGF2 in a fibronectin-coated culture dish, and MSCs were induced.

After the MSCs were induced, the 9^{th} passage cells, cultured for one month, were infected with one of the following viral vectors.
·control (no infection or an empty viral vector)
·c-MYC alone
·c-MYC+BMI1(MB)
·c-MYC+BMI1+BCLXL(MBX)
·c-MYC+BMI1+BCLXL+shp53
·c-MYC+BMI1+BCLXL+shp53+shp21
·shp53(p53KD)
·shp21(p21KD)
alfa MEM+10%FCS+5 ng/ml bFGF was used as the basal medium for iPS-MSC.

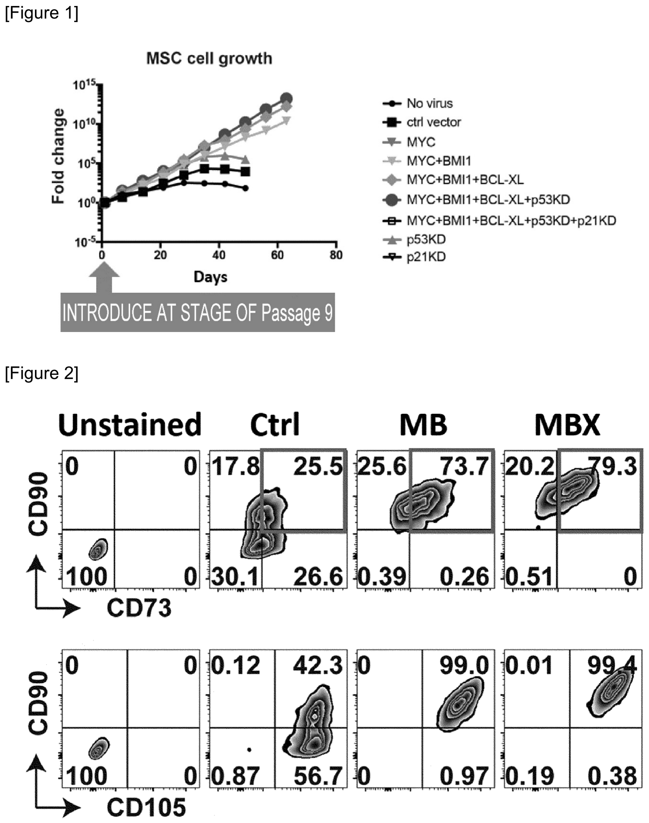

In the forced expression vectors for MYC, BMI1, and BCLXL, a system that induces expression with doxycycline (dox on system) was used, 1 µg/ml of docycycline was added to the culture solution immediately after infection, and the number of live cells was counted. The results are shown in Figure 1. As shown in Figure 1, in the control (no infection or an empty viral vector), proliferation was gradually stopped. In the p53KD, transient proliferation was observed, but proliferation was gradually stopped similarly. In addition, in the forced expression of c-MYC alone and in the p21KD, proliferation stopped earlier than in the control condition. On the other hand, in the combination including MB (c-MYC+BMI1, c-MYC+BMI1+BCLXL, c-MYC+BMI1+BCLXL+shp53, c-MYC+BMI1+BCLXL+shp53+shp21), exponential proliferation was confirmed even over 60 days after infection.

In addition, the cells were stained with CD90-FITC, CD73-PE, and CD105-APC, which are representative markers for mesenchymal stromal cells, and analyzed using a flow cytometer. The results are shown in Figure 2. Interestingly, the proportion of the CD90+/CD73+/CD105+ cell population decreased in the cntrol group, but increased in the MB and MBX groups. In the cntrol group, the CD90+ cell population and the CD90- cell population were observed. CD90 is a representative marker for mesenchymal stromal cells, and as terminal differentiation from mesenchymal stromal cells progressed, they lost stemness and also lost the expression of CD90. These results suggest that, in the cntrol group, the cells gradually lost stemness with the passage and underwent terminal differentiation, and mesenchymal stromal cells (CD90-) appeared, but in the MB and MBX groups, the cells maintained stemness, and the majority of the cells were more primitive mesenchymal stromal cells (CD90+).

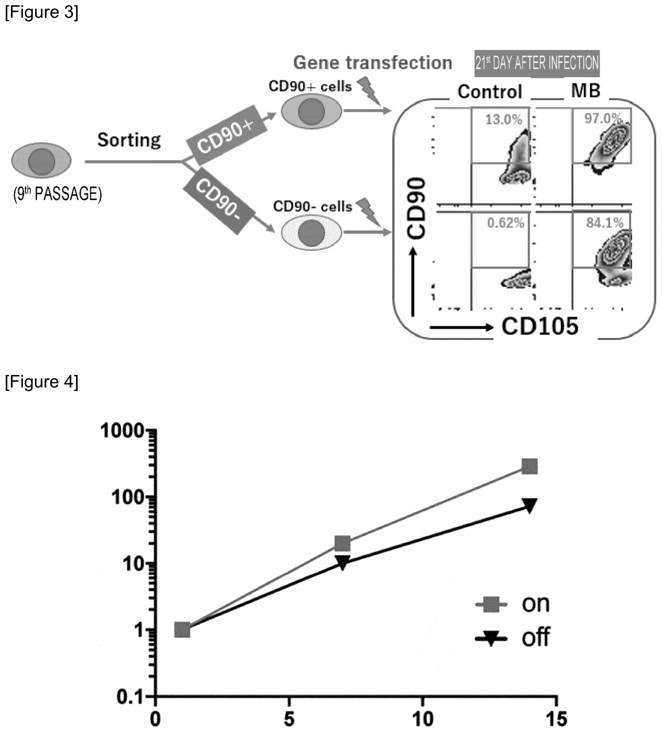

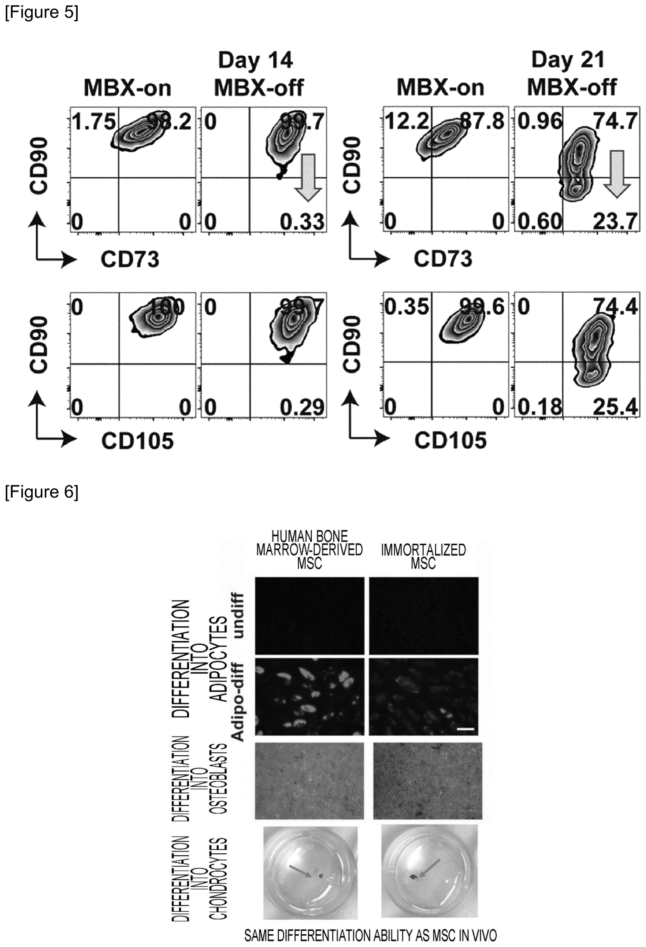

In addition, a cell population containing the 9^{th} passage differentiated cells (CD90-) was stained with CD90 antibodies, and the cells were then divided into the CD90+ fraction and the CD90- fraction and purified, a control (no infection) was prepared, and after MB infection, 1 µg/mL of doxycycline was added to both groups, and the cells were cultured and subjected to flow cytometry analysis on the 14^{th} and 21^{st} days after infection. The results are shown in Figures 3 and 5.

On the 21^{st} day after infection, CD105, CD90, and CD73 were analyzed by FACS, and in the control group, 13% of the CD90+ purified group maintained CD90+, but most of the cells were CD90- differentiated cells. In addition, most cells of the CD90- purified group were CD90- differentiated cells. The CD90+ fraction generated both the CD90+ fraction and the CD90- fraction, but the CD90- fraction did not generate the CD90+ fraction, suggesting that the CD90+ fraction was positioned upstream in the hierarchy. On the other hand, in the MB group, in both the CD90+ purified group and the CD90- purified group, most cell populations expressed CD90+. The CD90+CD73+CD105+ fraction was generated from both the CD90+ fraction and the CD90- fraction according to the forced expression of c-MYC+BMI1, indicating that c-MYC+BMI1 converted terminally differentiated mesenchymal stromal cells (CD90-) to more primitive mesenchymal stromal cells (CD90+) (reverting mesenchymal stromal cells into progenitor cells, progenitor cell reprogramming).

In addition, it was confirmed that, when Dox was turned off to stop the expression of MB or MBX, the proliferation rate slowed down (about 1/2 to 1/3 of that of when Dox was turned on), and CD90 expression was gradually lost (differentiated) (Figure 4). The introduction of MBX reverted the MSCs to those around Passage 0.

### Example 1-2: Inducing differentiation into chondrocytes, osteoblasts and adipocytes

MSCs (immortalized MSCs) having a proliferative ability established by forcibly expressing MB or MBX in iPS-derived MSCs according to Example 1-1 were induced to differentiate into adipocytes, osteoblasts, and chondrocytes, and the differentiation into each lineage was compared with that of human bone marrow-derived MSCs. Differentiation into adipocytes was performed using StemPro (trademark) Adipogenesis Differentiation Kit gibco (cat: A1007001), and confirmed by staining them with HCS (High-content screening) LipidTOX (trademark) Green neutral lipid stain (Invitrogen (Molecular Probes)) Cat: H34475. Differentiation into osteoblasts was performed using StemPro (trademark) Adipogenesis Differentiation Kit gibco (cat: A1007001), and confirmed by staining them with Alizarin Red S Staining Quantification Assay scien cell Research laboratories#8678. Differentiation into chondrocytes was performed using StemPro (trademark) Osteogenesis Differentiation Kit gibco (cat: A1007201), and confirmed by staining them with Alcian blue stain solution pH=2.5(Sigma)cat: A3157. The results are shown in Figure 6.

As a result, differentiation into any lineage was equivalent to that of human bone marrow-derived MSCs, indicating that the MSCs having a proliferative ability have the same differentiation ability as MSCs in vivo.

### Example 2: Vascular endothelial cells

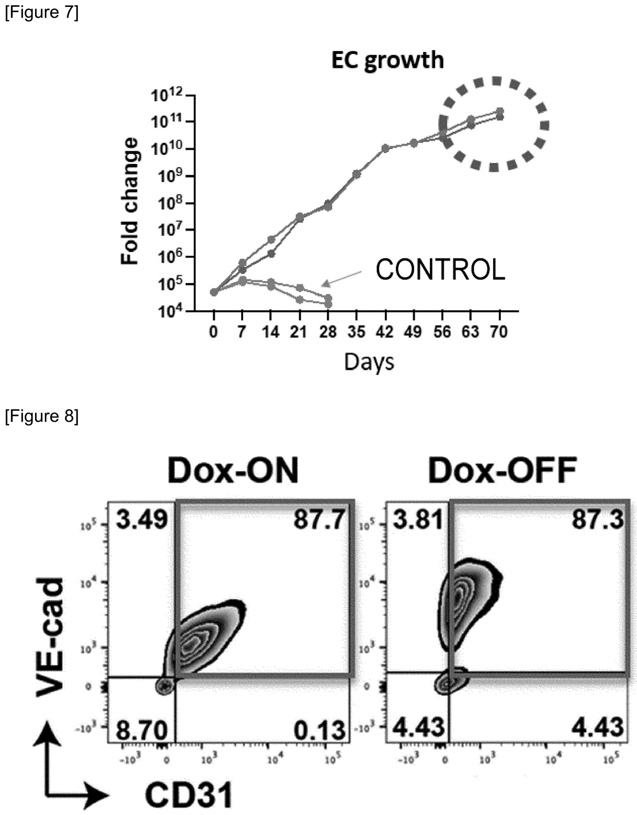

Using the method described in Takayama Blood 2008, 111(11): 5298-5306; Takayama JEM 2010, 207(13): 2817-2830, vascular progenitor cells were induced from human iPS cells by co-culturing with 10T1/2 cells in the presence of 20 ng/ml VEGF, and on the 10^{th} and 11^{th} day, CD34+ progenitor cells of vascular endothelial cells were sorted using FACS Arialll and infected with Dox-on c-MYC/Dox-on BMI1/Dox-on BCL-XL lentiviral vectors, culture was continued in the presence of 1 µg/ml of doxycycline and 100 ng/ml VEGF, and the number of cells was counted. In the dox-on state, the cell proliferation was significantly increased compared to the control (no virus infection) (Figure 7). In addition, immunostaining for the cell surface marker VE-cadherin was performed in the dox-on state and in the matured state after 7-day dox off.

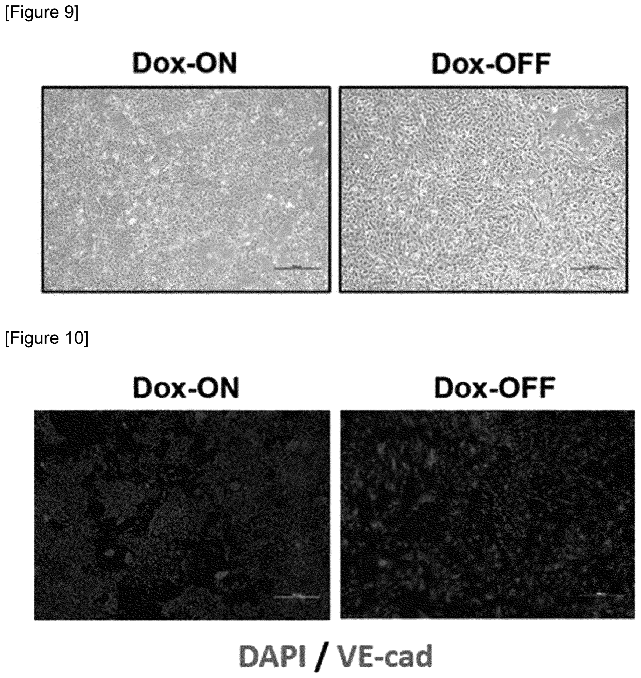

As a result, it was confirmed that the dox-off cells exhibited a higher expression level of VE-cadherin and were differentiated into vascular endothelial cells (Figures 8 to 10).

In addition, qPCR was used to evaluate gene expression of vascular endothelial cell differentiation markers ENG (CD105), MCAM (CD146), and PECAM1 in human iPS, imVEC-dox on, imVEC-dox off, human aortic endothelial cells (HAECs), and human umbilical vein endothelial cells (HUVECs).

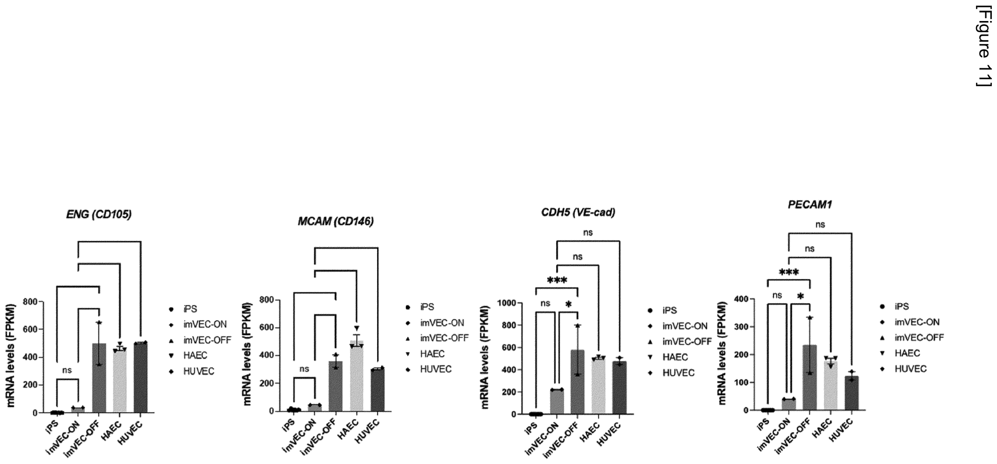

As a result, it was confirmed that cells obtained from dox-off human imVEC expressed the same level of vascular endothelial cell differentiation markers as HAECs and HUVECs (Figure 11).

### Example 3: Vascular smooth muscle cells

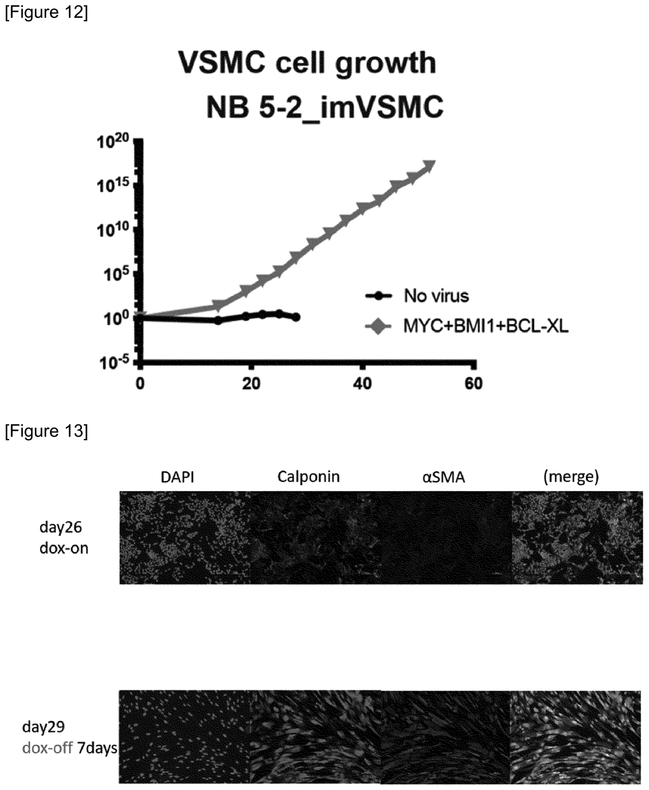

Using the method described in Takayama Blood 2008, 111(11): 5298-5306; Takayama JEM 2010, 207(13): 2817-2830, vascular progenitor cells were induced from human iPS cells by co-culturing with 10T1/2 cells in the presence of 20 ng/ml VEGF, and on the 10^{th} and 11^{th} day, CD34-/VEGFR<KDR>+ vascular smooth muscle progenitor cells were sorted using FACS Arialll and infected with Dox-on c-MYC/Dox-on BMI1/Dox-on BCL-XL lentiviral vectors, culture was continued in the presence of 1 µg/ml of doxycycline, and the number of cells was counted. In the dox-on state, the cell proliferation was significantly increased compared to the control (no virus infection) (Figure 12).

In addition, cells on the 26^{th} day after culture (dox-on progenitor cell stage) and cells left in the dox-off state for one week from day 22 (day 29) were immunostained with the differentiation markers Calponin and αSMA for smooth muscle.

As a result, it was confirmed that, in the dox-off cells, the expression of vascular smooth muscle differentiation markers was enhanced (Figure 13).

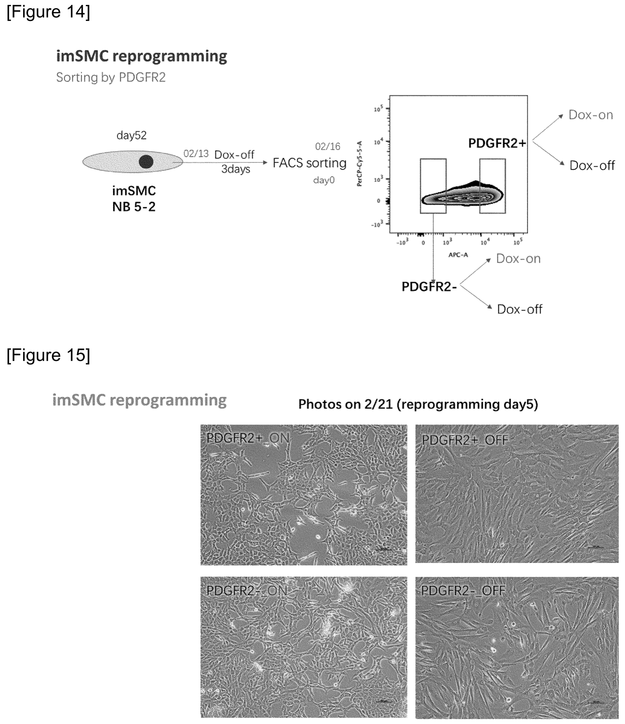

The immortalized vascular smooth muscle cells with improved proliferation by MBX introduction were left in the dox-off state (MBX off), incubated with PDGFR2-APC antibodies on the 3^{rd} day, then analyzed using FACS Canto II, and sorted into PDGFR2+ undifferentiated vascular smooth muscle cells and PDGFR2-differentiated vascular smooth muscle cells, and purified (Figure 14). Then, the cells were divided again into a group in which 1 µg/ml of doxycycline was added and a group in which the dox-off state was continued without change, and morphological observation, counting the number of cells, and confirmation of the cell surface marker (CD140b, KDR, and CD34) using FACS were performed. Specifically, a total of four groups below were tested.
I) PDGFR2+ undifferentiated vascular smooth muscle cells+dox on
II) PDGFR2+ undifferentiated vascular smooth muscle cells dox off
III) PDGFR2- differentiated vascular smooth muscle cells+dox on
IV) PDGFR2- differentiated vascular smooth muscle cells dox off

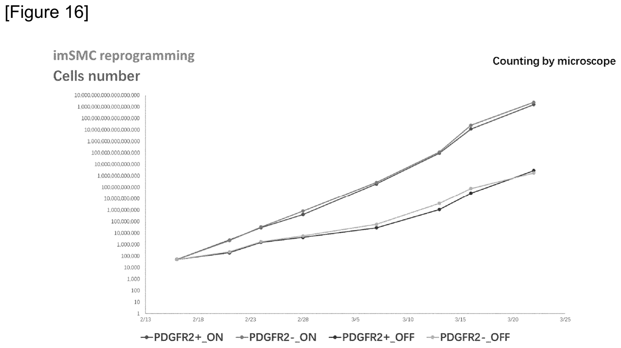

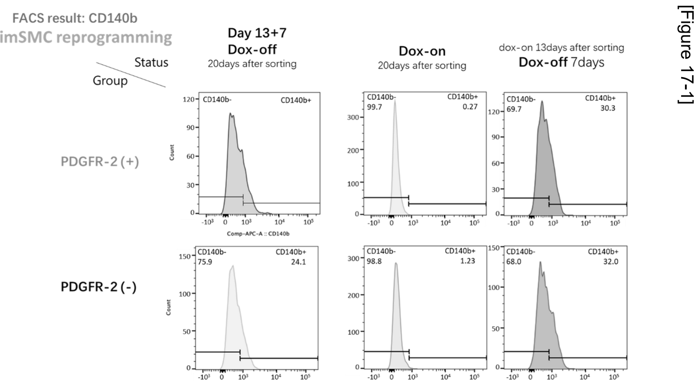

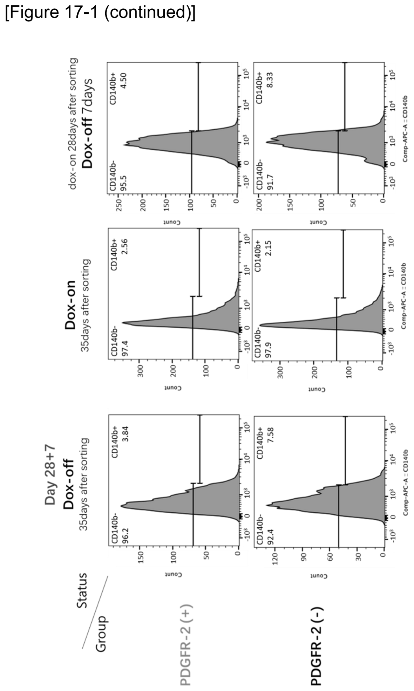

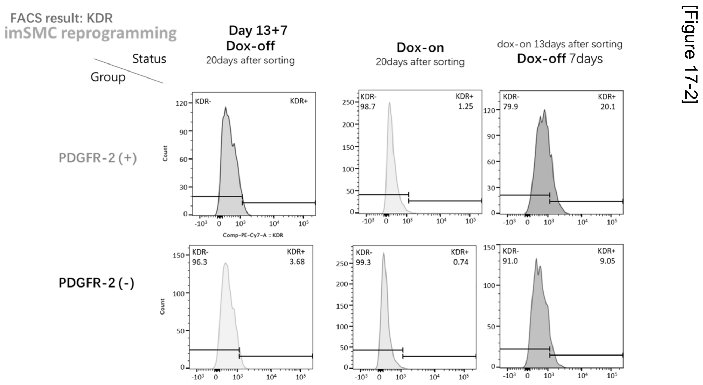

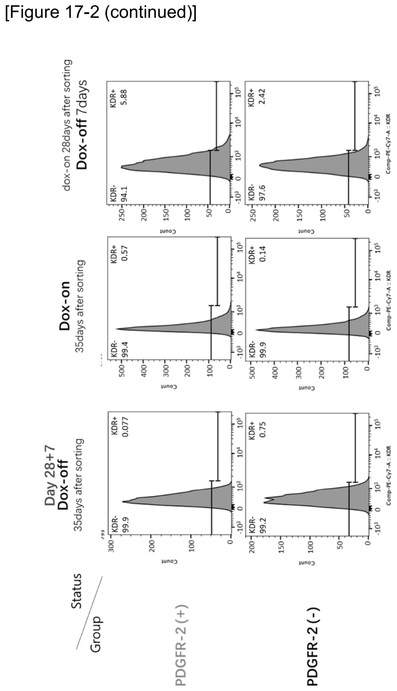

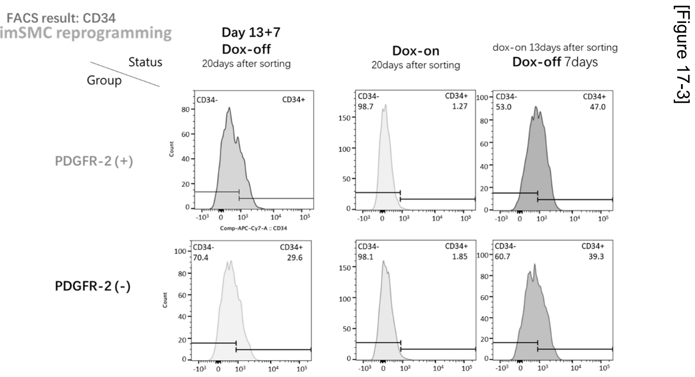

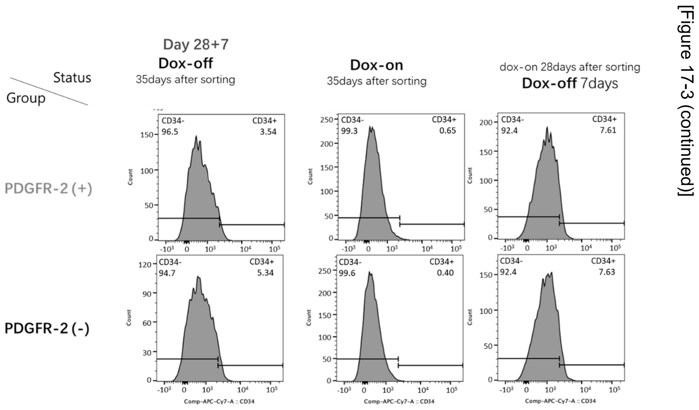

Figure 15 shows the results of morphological observation of the groups I) to IV) on the 5^{th} day after sorting, Figure 16 shows the results of the counted cell numbers, and Figures 17-1 to 17-3 show the confirmed results of the expression of cell surface markers. In the results of Figure 16 and Figures 17-1 to 17-3, in both the dox-on group I) PDGFR2+ undifferentiated vascular smooth muscle cells+dox on, and the group III) PDGFR2- differentiated vascular smooth muscle cells+dox on, compared to the dox-off group II) PDGFR2+ undifferentiated vascular smooth muscle cells dox off, and the group IV) PDGFR2- differentiated vascular smooth muscle cells dox off, the expression level of the surface markers CD140b, KDR, and CD34 was reduced, and the cell proliferation rate increased, indicating that the cells reverted to undifferentiated vascular smooth muscle cells. These results suggest that, when MBX was forcibly expressed in PDGFR2- differentiated vascular smooth muscle cells, the cells reverted to undifferentiated vascular smooth muscle cells having a proliferative ability (progenitor cells of vascular smooth muscle cells, PDGFR2+) (progenitor cell reprogramming). On the other hand, the results suggest that, when MBX expression in undifferentiated vascular smooth muscle cells (progenitor cells of vascular smooth muscle cells, PDGFR2+) that express MBX stopped, the cells differentiated into mature vascular smooth muscle cells (PDGFR2-).

In addition, after sorting, PDGFR2+ undifferentiated vascular smooth muscle cells and PDGFR2- differentiated vascular smooth muscle cells were cultured in the dox-on state for 28 days and then left in the dox-off for one week from day 29, and the cells were immunostained with the differentiation markers Calponin and αSMA for smooth muscle.

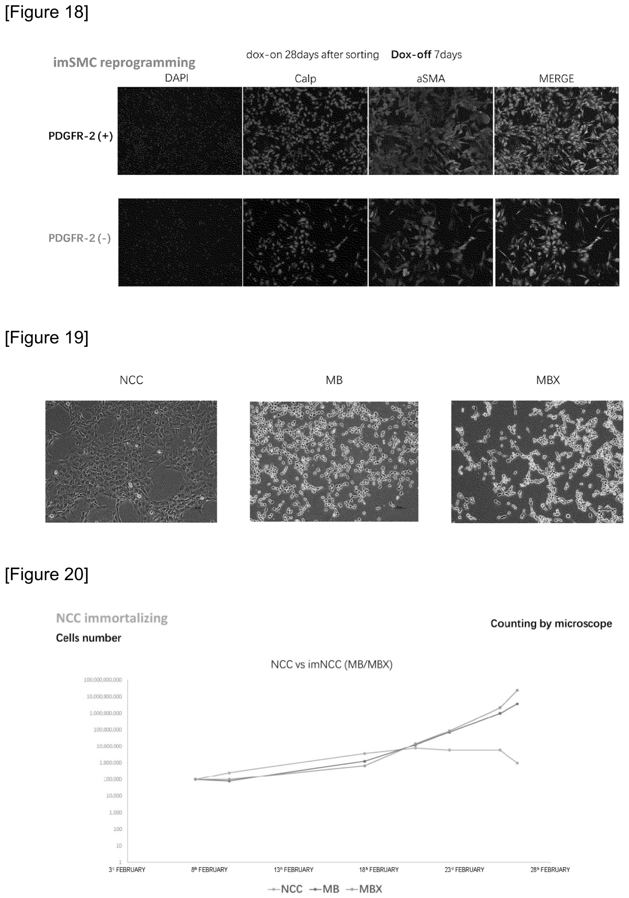

As a result, it was confirmed that, in both the PDGFR2+ undifferentiated vascular smooth muscle cells and the PDGFR2- differentiated vascular smooth muscle cells, the expression of smooth muscle differentiation markers was enhanced (Figure 18). In the PDGFR2- differentiated vascular smooth muscle cells in which MBX was forcibly expressed (reprogrammed progenitor cells), the expression of the smooth muscle differentiation markers was enhanced, suggesting that a favorable differentiation ability was maintained even after progenitor cell reprogramming.

### Example 4: Neural crest cells

According to the report of Fukuta et al. (PlosOne, 2014, 9(12): e112291), neural crest cells (NCCs) were induced from iPS cells in a Matrigel-coated culture dish in the presence of SB-431542 and CHIR99021 after 7 days of culture. Then, the medium was changed to an NCC medium including a CDMi medium containing EGF, FGF2, and SB-431542 (a CDMi medium was prepared based on an IMDM medium containing F-12 w, BSA, CD concentrated lipid, Apo-transferrin, 1-Thioglycerol, and P/S and Insulin), and in a fibronectin-coated culture dish, the NCCs were amplified.

The cultured NCCs were infected with c-MYC+BMI1 (MB) lentiviral vectors or c-MYC+BMI1+BCLXL(MBX) lentiviral vectors, culture was continued in the presence of 1 µg/ml of doxycycline, and morphological observation, counting the number of cells, and confirmation of the surface marker (CD271) using FACS were performed (Figures 19 to 23).

Figure 19 shows the results of morphological observation of the control group (no infection or an empty viral vector), and NCCs infected with c-MYC+BMI1 (MB) lentiviral vectors, or c-MYC+BMI1+BCLXL(MBX) lentiviral vectors 10 days after sorting. In addition, as shown in Figure 20, it was confirmed that cell proliferation gradually stopped in the control group, but cells proliferated exponentially in the NCCs infected with c-MYC+BMI1(MB) lentiviral vectors or c-MYC+BMI1+BCLXL(MBX) lentiviral vectors.

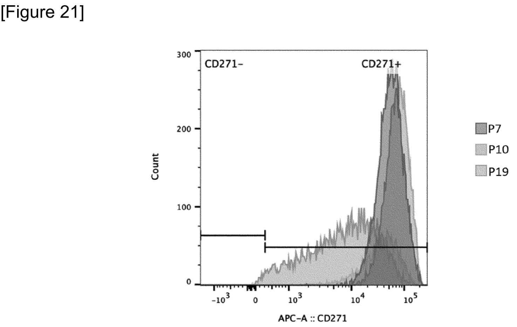

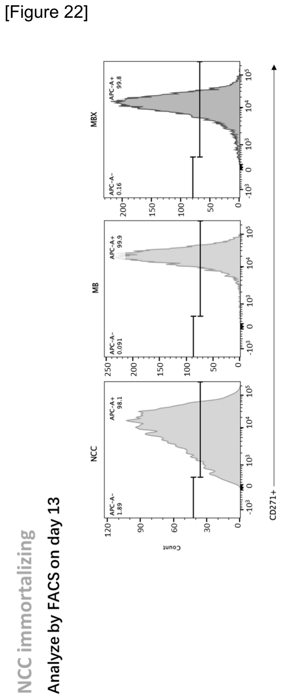

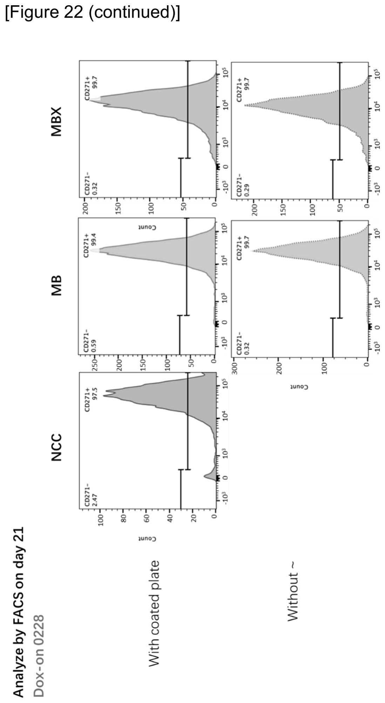

In addition, the expression of the cell surface marker CD271 for neural crest cells was analyzed using a flow cytometer. The results are shown in Figures F and G. As the neural crest cells underwent terminal differentiation, they lost stemness and lost the expression of CD271. In the cntrol group, the expression level of CD271 decreased as the number of passages increased (Figure 21), but in the MB and MBX groups, the expression was restored (Figure 22). These results suggest that, in the control group, the cells gradually lost stemness with the passage and underwent terminal differentiation, but in the MB and MBX groups, the cells maintained stemness and undifferentiated NCCs were maintained.

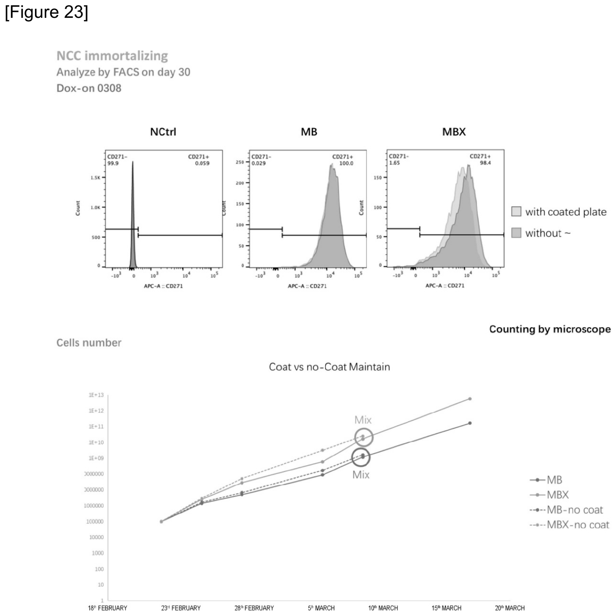

In addition, it was confirmed that the presence of a fibronectin coating on a culture dish had almost no effect on the number of cells in the MB and MBX groups and the expression of CD271 (Figure 23).

### Example 5: Lymphocytes

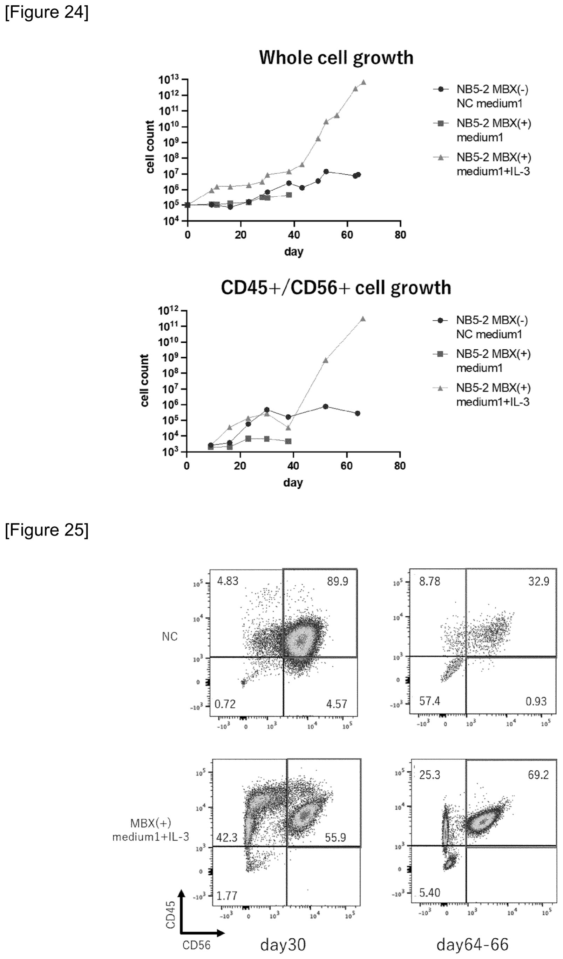

1 ml of 0.1% gelatin was added to each well of a 6-well plate and incubated at 37°C for 30 minutes. The gelatin was removed, and immortalized vascular endothelial cells were seeded at 2 to 3×10^{^5} cells/well in a basal medium (IMDM+15% FBS+10 µg/ml human insulin .5 µg/ml transferrin+5 ng/ml sodium selenite+2 mM L-glutamine+0.45 mM α-monothioglycerol+50 µl/ml ascorbic acid+20 ng/ml VEGF). The immortalized vascular endothelial cells used in this test were vascular endothelial cells obtained when vascular endothelial cells whose proliferation was improved by introducing MBX according to Example 2 were left in the dox-off state and cultured for 7 days and matured, and radiation exposure was performed to stop proliferation. The cells were cultured at 37°C and CO₂ 5.0%. The next day, the medium was removed, and iPS-derived human hematopoietic stem cells were seeded at 1×10^{^5} cells/well in a basal medium for iPS cell-derived hematopoietic progenitor cells (IMDM+15% FBS+10 µg/ml human insulin+5.5 µg/ml transferrin+5 ng/ml sodium selenite+2 mM L-glutamine+0.45 mM α-monothioglycerol+50 µl/ml ascorbic acid+50 ng/ml human FIt3L+50 ng/ml human SCF+20 ng/ml IL-7+20 ng/ml IL-15). On the same day, doxycycline-inducible c-MYC+BMI1+BCLXL(MBX) viral vectors were added, and 1 µg/ml of doxycycline was additionally added. The basic cytokine concentrations were hFlt3L 50 ng/ml+hSCF 50 ng/ml+IL-7 20 ng/ml+IL-15 20 ng/ml, and in the IL-3-addition group, 20 ng/ml of IL-3 was additionally administered for the first two weeks only, and all cells were cultured at 37°C and CO₂ 5.0%. Thereafter, the cells were cultured while replacing half of the medium every 2 or 3 days. In addition, the cells were stained with CD45-BV421, CD56-FITC, and CD19-PC7, and CD45+/CD56+ cells and CD45+/CD19+ cells were analyzed using FACS Cantoll (Figures 24 to 27). CD45-BV421 and CD56-FITC are cell surface markers for natural killer cells, and CD45-BV421 and CD19-PC7 are cell surface markers for B cells.

As shown in Figure 24, the total number of cells and the number of CD45+/CD56+ cells increased exponentially for two months or longer in the MBX(+)+IL-3 group, but the increase gradually stopped after the 50^{th} day of culture in the MBX(-) group (no infection or an empty viral vector). In the MBX(+) group, the culture was discontinued on the 38^{th} day because proliferation of CD45+/CD56+ cells was limited.

In addition, as shown in Figure 25, in the MBX(-) group, the proportion of the CD45+/CD56+ cell population decreased to 89.9% on the 30^{th} day after culture, and 32.9% day 64, and on the other hand, in the MBX(+)+IL-3 group, the proportion was 55.9% on day 30 and 69.2% on day 66, confirming that the proportion of the cell population was still high at 50% or more even over 66 days.

Therefore, in the MBX(+)+IL-3 group, CD45+/CD56+ cells having a high proliferative ability were obtained, and a large number of immortalized natural killer cells were successfully produced with high efficiency.

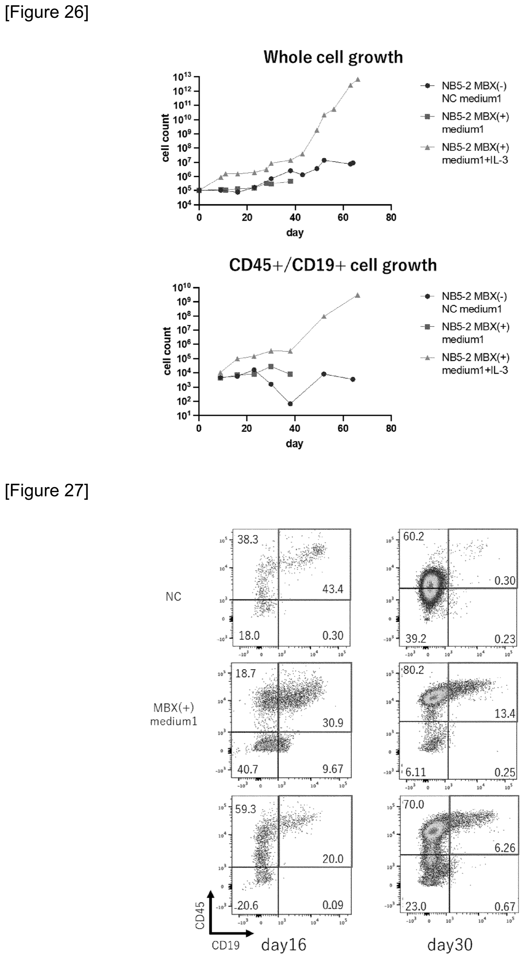

In addition, as shown in Figures 26 and 27, the CD45+/CD19+ cell population also appeared, the number of CD45+/CD19+ cells increased exponentially for two months or longer in the MBX(+)+IL-3 group, but the increase gradually stopped after the 50^{th} day of culture in the MBX(-) group (no infection or an empty viral vector). In the MBX(+) group, the culture was discontinued on the 38^{th} day because proliferation of CD45+/CD19+ cells was limited.

Therefore, in the MBX(+)+IL-3 group, CD45+/CD19+ cells having a high proliferative ability were obtained, and a large number of immortalized B cells were successfully produced with high efficiency.

Based on the above results, it can be understood that, when c-MYC, BMI1, and BCLXL(MBX) were forcibly expressed, IL-3 was added, and culture was performed, it was possible to obtain immortalized lymphocyte cells such as immortalized natural killer cells and immortalized B cells.

### Example 6: Myelocytes and megakaryocytes

An experiment was performed using megakaryocytes and myelocytes induced from human iPS cells whose proliferation had already been improved by introducing MBX. For the culture, IMDM+ was used as the basal medium. Here, the megakaryocytes were cultured in 50 ng/ml SCF and 50 ng/mL TPO, and the myelocytes were cultured in M-CSF 50 ng/mL, GM-CSF 50 ng/mL, G-CSF 25 ng/mL, IL-3 25 ng/mL, SCF 25 ng/mL, and TPO 5 ng/mL.

### Megakaryocytes

In the dox-off (MBX off) state, on the 3^{rd} day, CD41+CD42b- undifferentiated megakaryocytes, and CD41+CD42b+ differentiated megakaryocytes were sorted and purified using FACS arialll, and then divided again into a group in which 1 µg/ml of doxycycline was added and a group in which the dox-off state was continued without change, and counting the number of cells, and confirmation of the surface marker using FACS were performed. Specifically, a total of four groups below were tested.
I) CD41+CD42b- undifferentiated megakaryocytes+dox on
II) CD41+CD42b- undifferentiated megakaryocytes dox off
III) CD41+CD42b+ differentiated megakaryocytes+dox on
IV) CD41+CD42b+ differentiated megakaryocytes dox off

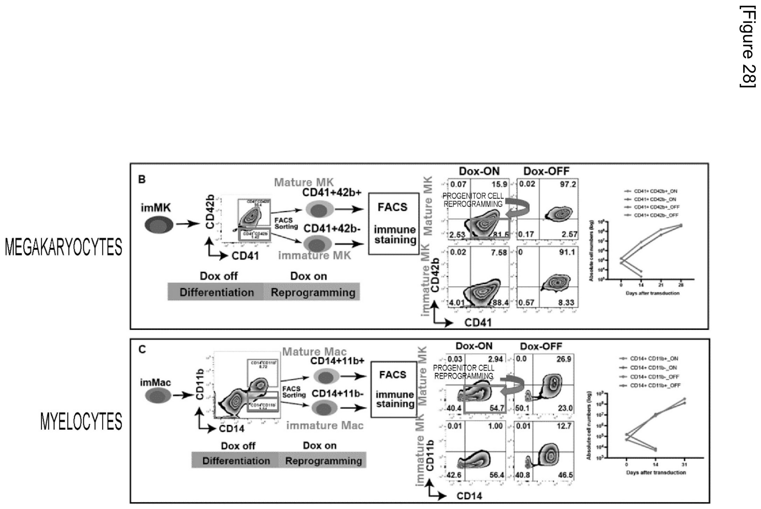

The results are shown in Figure 28. In both the group I) CD41+CD42b-undifferentiated megakaryocytes+dox on and the group III) CD41+CD42b+ differentiated megakaryocytes+dox on, in the doxycycline+ group, cells proliferated exponentially, and all cells reverted to CD41+CD42b- undifferentiated megakaryocytes. On the other hand, in the dox-off group II) CD41+CD42b-undifferentiated megakaryocytes dox off and group IV) CD41+CD42b+ differentiated megakaryocytes dox off, the cells stopped proliferating and become CD41+CD42b+ differentiated megakaryocytes. These results suggest that, when MBX was forcibly expressed in differentiated megakaryocytes (CD41+CD42b+), the cells reverted to undifferentiated megakaryocytes having a proliferative ability (megakaryocyte progenitor cells, CD41+CD42b-) (progenitor cell reprogramming). On the other hand, the results suggest that, when MBX expression in undifferentiated megakaryocytes (megakaryocyte progenitor cells, CD41+CD42b-) that express MBX stopped, the cells lost their proliferative ability and differentiated into mature megakaryocytes (CD41+CD42b+).

### Myelocytes

In the dox-off (MBX off) state, on the 3^{rd} day, CD14+CD11b- undifferentiated macrophage and CD14+CD11b+ differentiated macrophages were sorted and purified using FACS arialll, and then divided again into a group in which 1 µg/ml of doxycycline was added and a group in which the dox-off state was continued without change, and counting the number of cells, and counting the number of cells, and confirmation of the surface marker using FACS were performed. Specifically, a total of four groups below were tested.
I) CD14+CD11b- undifferentiated macrophages+dox on
II) CD14+CD11b- undifferentiated macrophages dox off
III)CD14+CD11b+ differentiated macrophages+dox on
IV) CD14+CD11b+ differentiated macrophages dox off

The results are shown in the lower part of Figure 28. In both the group I) CD14+CD11b- undifferentiated macrophages+dox on, and the group III) CD14+CD11b+ differentiated macrophages+dox on, in the doxycycline+ group, cells proliferated exponentially, and all cells reverted to CD14+CD11b- undifferentiated macrophages.

On the other hand, in the dox-off group II) CD14+CD11b- undifferentiated macrophage dox off and group IV) CD14+CD11b+ differentiated macrophages dox off, the cell stopped proliferating and became CD14+CD11b+ differentiated macrophages. These results suggest that, when MBX was forcibly expressed in differentiated macrophages (CD14+CD11b+), the cells reverted to undifferentiated macrophages having a proliferative ability (CD14+CD11b-) (progenitor cell reprogramming). On the other hand, the results suggest that, when MBX expression in undifferentiated macrophages (CD14+CD11b-) that express MBX stopped, the cells lost their proliferative ability and differentiated into mature macrophages (CD14+CD11b+).

### Example 7-1: Co-culture with immortalized vascular endothelial cells

It is known that a notch signal, which is transmitted through the interaction between Notch and Notch ligands (Delta-like4 (hereinafter referred to as DLL4), Jagged 1, etc.), is necessary for cell differentiation.

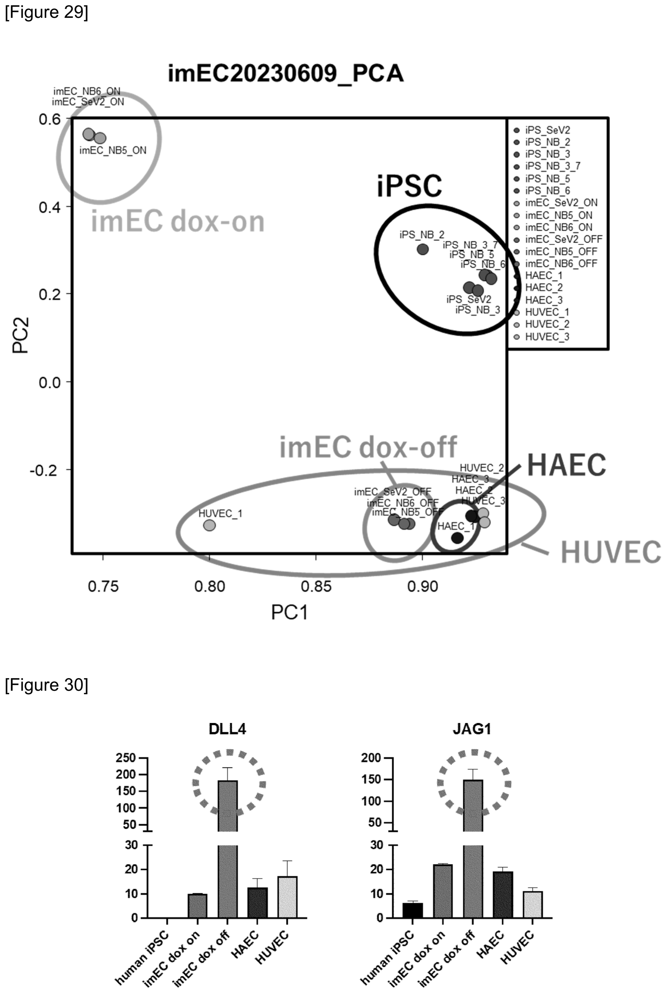

In addition, dox-on immortalized vascular endothelial cells, and dox-off immortalized vascular endothelial cells, human iPS cells, human aortic endothelial cells (HAEC), and human umbilical vein endothelial cells (HUVEC) were subjected to principal component analysis (PCA), and as a result, it was found that the principal components of the dox-off immortalized vascular endothelial cells were similar to the principal components of the human aortic endothelial cells (HAEC) and the human umbilical vein endothelial cells (HUVEC) (Figure 29). In addition, it was found that DLL4 and Jagged 1 were more highly expressed in the dox-off immortalized vascular endothelial cells than in the dox-on immortalized vascular endothelial cells, human iPS cells, human aortic endothelial cells (HAEC), and human umbilical vein endothelial cells (HUVEC) (Figure 30).

Therefore, the effect of immortalized vascular endothelial cells on differentiation of hematopoietic stem cells or hematopoietic progenitor cells into lymphocytes was studied.

### Example 7-2: Co-culture of immortalized vascular endothelial cells and CD34-positive umbilical cord blood cells

The immortalized vascular endothelial cells used in this test were vascular endothelial cells obtained when vascular endothelial cells whose proliferation was improved by introducing MBX according to Example 2 were left in the dox-off state and cultured for 7 days and matured, and radiation exposure was performed to stop proliferation.

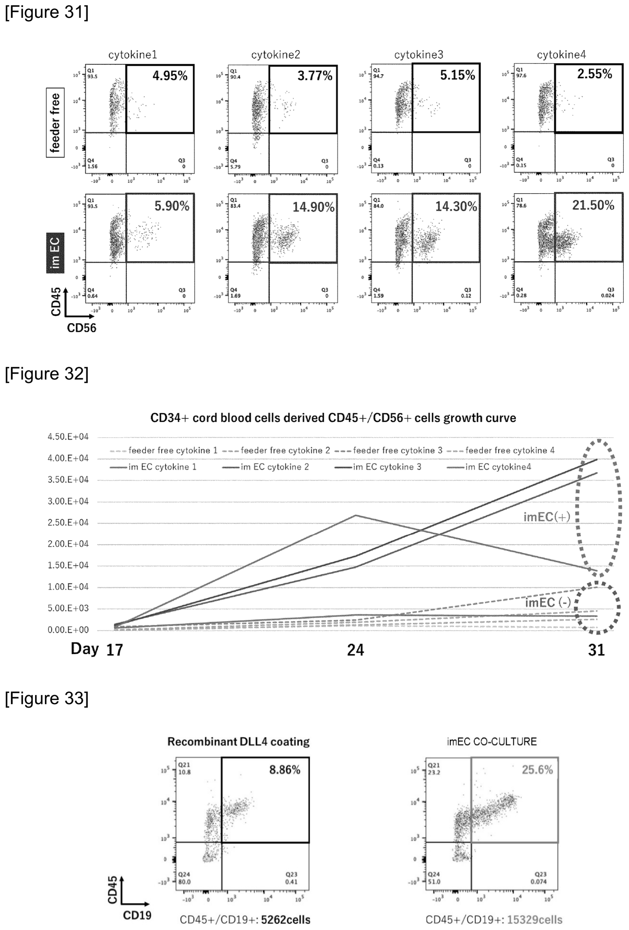

1 ml of 0.1% gelatin was added to each well of a 6-well plate and incubated at 37°C for 30 minutes. The gelatin was removed, and immortalized vascular endothelial cells were seeded at 2 to 3×10^{^5} cells/well in a basal medium, and cultured at 37°C and CO₂ 5.0%. The next day, the medium was removed, and CD34-positive umbilical cord blood cells were seeded at 5×10^{^3} cells/well in a basal medium for CD34-positive umbilical cord blood cell-derived hematopoietic progenitor cells (X-VIVO10+10% BSA). In addition, in wells in which immortalized vascular endothelial cells were not seeded (control, feeder free), CD34-positive umbilical cord blood cells were seeded at 5×10^{^3} cells/well in a basal medium for CD34-positive umbilical cord blood cell-derived hematopoietic progenitor cells. Regarding cytokine conditions, four types of cytokines (Cytokines 1 to 4) were prepared, and all cells were cultured at 37°C and CO₂ 5.0%. Thereafter, the cells were cultured while replacing half of the medium every 2 or 3 days, and on the 24^{th} day after culture, the cells were stained with CD45-BV421 and CD56-FITC, which are cell surface markers for natural killer cells, and CD45+/CD56+ cells were analyzed using FACS Canto II (Figures 31 and 32). The conditions for Cytokines 1 to 4 are shown below.
·Cytokine 1:
   X-VIVO10+10% BSA+50 ng/ml human Flt3+50 ng/ml human SCF+20 ng/ml IL-7+20 ng/ml IL-15
·Cytokine 2:
   X-VIVO10+10% BSA+50 ng/ml human Flt3+50 ng/ml human SCF+20 ng/ml IL-7+20 ng/ml IL-15+20 ng/ml IL-2+50 ng/ml TPO
·Cytokine 3:
   X-VIVO10+10% BSA+50 ng/ml human Flt3+50 ng/ml human SCF+20 ng/ml IL-7+20 ng/ml IL-15+20 ng/ml IL-2+50 ng/ml TPO+10 pg/ml GM-CSF+250 pg/ml G-CSF+50 pg/ml IL-6
·Cytokine 4:
   day0-9; X-VIVO10+10% BSA+50 ng/ml human Flt3+50 ng/ml human SCF+20 ng/ml IL-7+50 ng/ml TPO+10 pg/ml GM-CSF+250 pg/ml G-CSF+50 pg/ml IL-6
   day9-14; X-VIVO10+10% BSA+50 ng/ml human Flt3+50 ng/ml human SCF+20 ng/ml IL-7+20 ng/ml IL-15+10 pg/ml GM-CSF+250 pg/ml G-CSF+50 pg/ml IL-6
   day14-; X-VIVO10+10% BSA+50 ng/ml human SCF+20 ng/ml IL-7+20 ng/ml IL-15+20 ng/ml IL-2+10 pg/ml GM-CSF+250 pg/ml G-CSF+50 pg/ml IL-6

As shown in Figure 31, under all conditions for Cytokines 1 to 4, the CD45+/CD56+ cell population appeared, and the proportion of the CD45+/CD56+ cell population was higher when the cells were co-cultured with immortalized vascular endothelial cells (im EC) than when the cells were not co-cultured with immortalized vascular endothelial cells (feeder free).

In addition, as shown in Figure 32, focusing on the absolute number of cells, comparing cells cultured under the same cytokine conditions (for example, im EC cytokine3 and feeder free cytkine2), the number of CD45+/CD56+ cells was larger when the cells were co-cultured with immortalized vascular endothelial cells.

Based on the above results, it can be understood that co-culturing together with immortalized vascular endothelial cells can promote differentiation of hematopoietic stem cells or hematopoietic progenitor cells into natural killer cells. In addition, it can be understood that co-culturing can promote differentiation into lymphoid progenitor cells, which are intermediates before differentiation into natural killer cells.

### Example 7-3: Co-culture of immortalized vascular endothelial cells or gene recombinant DLL4 and iPS cell-derived human hematopoietic stem cells

The immortalized vascular endothelial cells used in this test were vascular endothelial cells obtained when vascular endothelial cells whose proliferation was improved by introducing MBX according to Example 2 were left in the dox-off state and cultured for 7 days and matured, and radiation exposure was performed to stop proliferation.

1 ml of 0.1% gelatin was added to each well of a 6-well plate and incubated at 37°C for 30 minutes. The gelatin was removed, and immortalized vascular endothelial cells were seeded at 2 to 3×10^{^5} cells/well in a basal medium (IMDM+15% FBS+10 µg/ml human insulin+5.5 µg/ml transferrin+5 ng/ml sodium selenite+2 mM L-glutamine+0.45 mM α-monothioglycerol+50 µl/ml ascorbic acid+20 ng/ml VEGF), and cultured at 37°C and CO₂ 5.0%. In addition, gene recombinant DLL4 was seeded in a separate well and refrigerated at 4°C. The next day, the medium was removed, and iPS-derived human hematopoietic stem cells were seeded at 8.4×10^{^4} cells/well in a basal medium for iPS cell-derived hematopoietic progenitor cells (IMDM+15% FBS+10 µg/ml human insulin+5.5 µg/ml transferrin+5 ng/ml sodium selenite+2 mM L-glutamine+0.45 mM α-monothioglycerol+50 µl/ml ascorbic acid+10 ng/ml human FIt3L+50 ng/ml human SCF+5 ng/ml IL-7+30 ng/ml TPO). Doxycycline-inducible c-MYC+BMI1+BCLXL(MBX) viral vectors were added, 1 µg/ml of doxycycline was additionally added, and all cells were cultured at 37°C and CO₂ 5.0%. Thereafter, the cells were cultured while replacing half of the medium every 2 or 3 days. Then, the cells were stained with CD45-BV421 and CD19-PC7, which are cell surface markers for B cells, and CD45+/CD19+ cells were analyzed using FACS Canto II (Figure 33).

As shown in Figure 33, the proportion of the CD45+/CD19+ cell population and the absolute number of cells were larger when the cells were co-cultured with immortalized vascular endothelial cells (im EC) than when the cells were co-cultured with gene recombinant DLL4. Therefore, it is conceivable that the immortalized vascular endothelial cells could highly express other Notch ligands such as Jagged 1 in addition to DLL4.

Based on the above results, it can be understood that co-culturing together with immortalized vascular endothelial cells can further promote differentiation of hematopoietic stem cells or hematopoietic progenitor cells into lymphoid cells such as B cells than co-culturing with gene recombinant DLL4. In addition, it can be understood that co-culturing together can further promote differentiation into lymphoid progenitor cells, which are intermediates before differentiation into B cells.

### Example 7-4: Co-culture of immortalized vascular endothelial cells and iPS cell-derived human hematopoietic stem cells after IL-3 was administered

The immortalized vascular endothelial cells used in this test were vascular endothelial cells obtained when vascular endothelial cells whose proliferation was improved by introducing MBX according to Example 2 were left in the dox-off state and cultured for 7 days and matured, and radiation exposure was performed to stop proliferation.

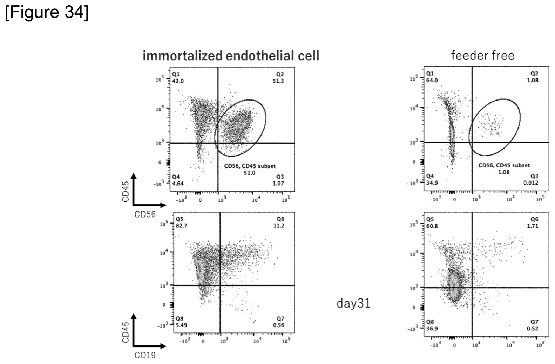

1 ml of 0.1% gelatin was added to each well of a 6-well plate and incubated at 37°C for 30 minutes. The gelatin was removed, and immortalized vascular endothelial cells were seeded at 2 to 3×10^{^5} cells/well in a basal medium (IMDM+15% FBS+10 µg/ml human insulin+5.5 µg/ml transferrin+5 ng/ml sodium selenite+2 mM L-glutamine+0.45 mM α-monothioglycerol+50 µl/ml ascorbic acid+20 ng/ml VEGF) and cultured at 37°C and CO₂ 5.0%. The next day, the medium was removed, and iPS-derived human hematopoietic stem cells were seeded at 8.4×10^{^4} cells/well in a basal medium for iPS cell-derived hematopoietic progenitor cells (IMDM+15% FBS+10 µg/ml human insulin+5.5 µg/ml transferrin+5 ng/ml sodium selenite+2 mM L-glutamine+0.45 mM α-monothioglycerol+50 µl/ml ascorbic acid+50 ng/ml human FIt3L+50 ng/ml human SCF+20 ng/ml IL-7+20 ng/ml IL-15). Doxycycline-inducible c-MYC+BMI1+BCLXL(MBX) viral vectors were added, 1 µg/ml of doxycycline was additionally added, and all cells were cultured at 37°C and CO₂ 5.0%. 20 ng/ml of IL-3 was administered for the first week only. Thereafter, the cells were cultured while replacing half of the medium every 2 or 3 days. Then, the cells were stained with CD45-BV421, CD56-FITC, and CD19-PC7, and CD45+/CD56+ cells and CD45+/CD19+ cells were analyzed using FACS Canto II (Figure 34). CD45-BV421 and CD56-FITC are cell surface markers for natural killer cells, and CD45-BV421 and CD19-PC7 are cell surface markers for B cells. The compositions of the mediums used for culture are shown below.

As shown in Figure 34, the proportions of the CD45+/CD56+ cell population and the CD45+/CD19+ cell population were higher when the cells were co-cultured with immortalized vascular endothelial cells (im EC) than when the cells were not co-cultured with immortalized vascular endothelial cells (feeder free).

Based on the above results, it can be understood that co-culturing together with immortalized vascular endothelial cells can promote differentiation of hematopoietic stem cells or hematopoietic progenitor cells into natural killer cells or B cells. In addition, it can be understood that co-culturing together can promote differentiation into lymphoid progenitor cells, which are intermediates before differentiation into natural killer cells or B cells.

## Claims

1. A method for regulating the degree of cell differentiation, comprising a step of forcibly expressing the MYC family gene and the BMI1 gene in cells selected from the group consisting of (i) mesenchymal stromal cells, vascular endothelial cells, smooth muscle cells, neural crest cells, and lymphocytes having any degree of differentiation, and (ii) megakaryocytes and myelocytes having a high degree of differentiation.

2. The method according to claim 1, wherein the cells are mesenchymal stromal cells having any degree of differentiation.

3. The method according to claim 1, wherein the cells are mesenchymal stromal cells that do not express a cell surface marker CD90.

4. The method according to claim 1, wherein the cells are mesenchymal stromal cells that express a cell surface marker CD90.

5. The method according to claim 1, wherein the cells are vascular endothelial cells having any degree of differentiation.

6. The method according to claim 1, wherein the cells are smooth muscle cells having any degree of differentiation.

7. The method according to claim 1, wherein the cells are vascular smooth muscle cells having any degree of differentiation.

8. The method according to claim 1, wherein the cells are vascular smooth muscle cells that express a cell surface marker CD140b, KDR, or CD34.

9. The method according to claim 1, wherein the cells are neural crest cells having any degree of differentiation.

10. The method according to claim 1, wherein the cells are lymphocytes having any degree of differentiation.

11. The method according to claim 1, wherein the cells are megakaryocytes that express cell surface markers CD41 and CD42b.

12. The method according to claim 1, wherein the cells are myelocytes that express cell surface markers CD14 and CD11b.

13. The method according to claim 1, further comprising a step of inhibiting the expression of the MYC family gene and the BMI1 gene or the function of expression products thereof.

14. The method according to claim 1, further comprising a step of forcibly expressing the BCL-XL gene.

15. The method according to claim 1, further comprising a step of inhibiting the expression of the BCL-XL gene or the function of expression products thereof.

16. The method according to claim 1, further comprising a step of inhibiting the expression of at least one of the CDKN1A gene and the P53 gene or the function of expression products thereof.

17. A method for producing progenitor cells of mesenchymal stromal cells, vascular endothelial cells, smooth muscle cells, neural crest cells, lymphocytes, megakaryocytes, or myelocytes, comprising a step of culturing cells obtained by the method according to claim 1.

18. A method for producing differentiated cells of mesenchymal stromal cells, vascular endothelial cells, smooth muscle cells, neural crest cells, lymphocytes, megakaryocytes, or myelocytes, comprising a step of differentiating cells obtained by the method according to claim 1.

19. Cells obtained by the method according to any one of claims 1 to 18.

20. A pharmaceutical composition comprising cells obtained by the method according to any one of claims 1 to 18.

21. A method for treating or preventing a disease, comprising administering cells obtained by the method according to any one of claims 1 to 18 to a patient in need thereof.

22. A cell differentiation regulating agent for cells selected from the group consisting of (i) mesenchymal stromal cells, vascular endothelial cells, smooth muscle cells, neural crest cells, and lymphocytes having any degree of differentiation, and (ii) megakaryocytes and myelocytes having a high degree of differentiation, comprising molecules that forcibly express the MYC family gene and the BMI1 gene as an active component.

23. A method for producing mesenchymal stromal cells having a proliferative ability, comprising a step of forcibly expressing the MYC family gene and the BMI1 gene in mesenchymal stromal cells having any degree of differentiation.

24. The production method according to claim 23, wherein the mesenchymal stromal cells having any degree of differentiation are mesenchymal stromal cells that do not express a cell surface marker CD90.

25. The production method according to claim 23, wherein the mesenchymal stromal cells having any degree of differentiation are mesenchymal stromal cells that express a cell surface marker CD90.

26. The production method according to any one of claims 23 to 25, wherein the mesenchymal stromal cells having a proliferative ability express a cell surface marker CD90.

27. A method for producing vascular endothelial cells having a proliferative ability, comprising a step of forcibly expressing the MYC family gene and the BMI1 gene in vascular endothelial cells having any degree of differentiation.

28. A method for producing smooth muscle cells having a proliferative ability, comprising a step of forcibly expressing the MYC family gene and the BMI1 gene in smooth muscle cells having any degree of differentiation.

29. The production method according to claim 28, wherein the smooth muscle cells having any degree of differentiation are vascular smooth muscle cells.

30. The production method according to claim 29, wherein the vascular smooth muscle cells are vascular smooth muscle cells that express a cell surface marker CD140b, KDR, or CD34.

31. A method for producing neural crest cells having a proliferative ability, comprising a step of forcibly expressing the MYC family gene and the BMI1 gene in neural crest cells having any degree of differentiation.

32. A method for producing lymphocytes having a proliferative ability, comprising a step of forcibly expressing the MYC family gene and the BMI1 gene in lymphocytes having any degree of differentiation.

33. A method for producing megakaryocytes having a proliferative ability, comprising a step of forcibly expressing the MYC family gene and the BMI1 gene in megakaryocytes that express cell surface markers CD41 and CD42b.

34. A method for producing macrophages having a proliferative ability, comprising a step of forcibly expressing the MYC family gene and the BMI1 gene in macrophages that express cell surface markers CD14 and CD11b.

35. A method for promoting differentiation of iPS cells, ES cells, hematopoietic stem cells or hematopoietic progenitor cells into lymphocyte cells or lymphoid progenitor cells, comprising a step of co-culturing immortalized vascular endothelial cells, and iPS cells, ES cells, hematopoietic stem cells or hematopoietic progenitor cells.

36. The method according to claim 35, wherein the lymphocyte cells express cell surface markers CD45 and CD56 or CD19 and CD45.

37. The method according to claim 35 or 36, wherein the lymphocyte cells are natural killer cells or B cells.

38. The method according to claim 35 or 36, wherein the hematopoietic stem cells or hematopoietic progenitor cells are derived from umbilical cord blood or iPS cells.

39. The method according to claim 35 or 36, wherein immortalized vascular endothelial cells express a Notch ligand.

40. The method according to claim 39, wherein the Notch ligand is DLL4 and/or Jagged 1.
